# EUROPEAN PATENT APPLICATION

(11) **EP 1 489 071 A1**
(43) Date of publication of application: **22.12.2004**
(21) Application number: 03013842.4
(22) Date of filing: 18.06.2003
(51) Int. Cl.: C07D 217/06, C07D 401/06, C07D 409/06, C07D 405/06, C07D 413/06, A61K 31/47, A61P 11/06, A61P 13/00, A61P 37/00, A61P 25/00

(54) **N-substituted, 3,4-dihydro-1H-isoquinoline as potassium channels modulators**

(71) Applicant: 4SC AG, 82152 Martinsried (DE)
(72) Inventor: Garcia, Gabriel, Dr., 81375 München (DE); Saeb, Wael, Dr., 82152 Martinsried (DE); Kramer, Bernd, Dr., 52080 Aachen (DE); Rauer, Heiko, 81377 München (DE); Vincek, Adam, 74078 Heilbronn (DE)
(74) Representative: Strehl Schübel-Hopf & Partner

(57) **Abstract**

The invention relates to compounds having the Formula (I) or a salt, or a physiologically functional derivative, or a prodrug thereof, wherein 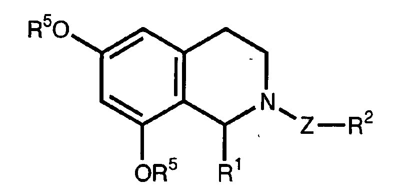 wherein
- Z: is carbonyl or sulfonyl;
- R¹: is alkyl, alkenyl, alkynyl, aryl, H, halogen, haloalkyl, haloalkoxy, hydroxyalkyl, cycloalkyl or heteroaryl;
- R²: is -CH₂-SO₂-alkyl, -CH₂-SO₂-cycloalkyl, -CH₂-SO₂-aryl, -CH₂-SO₂-heteroaryl, aryl, haloalkyl, haloalkoxy, hydroxyalkyl, cycloalkyl, heteroaryl or a linear or branched C₂-C₆-alkyl group, which can optionally be substituted by one or more substituents R³;
- R³: is H, alkyl, alkenyl, alkynyl, cycloalkyl, -CO₂R⁴, -CONHR⁴, -CR⁴O, -SO₂R⁴, -NR⁴-CO-R⁴, alkoxy, alkylthio, -OH, -SH, -O-aryl, -O-cycloalkyl, -S-aryl, -S-cycloalkyl, hydroxyalkyl, halogen, haloalkyl, haloalkoxy, CN, NO₂, hydroxyalkylamine, aminoalkyl, alkylamine, aryl or heteroaryl;
- R⁴: is H, halogen, alkyl, alkenyl, alkynyl, cycloalkyl, alkoxy, alkylthio, -O-aryl, -O-cycloalkyl, OH, SH, -S-aryl, -S-cycloalkyl, hydroxyalkyl, haloalkyl, haloalkoxy, hydroxyalkyl-amine, aminoalkyl, alkylamine, aryl or heteroaryl;
- R⁵: is alkyl, alkenyl, alkynyl.

## Description

The present invention relates to potassium channel modulating 6,8-dimethoxy isoquinolines derivatives. These compounds are useful in the treatment or alleviation of disorders and conditions associated with, or dependent on the membrane potential or conductance of cells in mammals, including a human. The present method also provides a method for the manufacture of medicaments and pharmaceutical compositions comprising the K⁺ channel modulating agents. The agents of the invention are useful for the treatment or alleviation of diseases, disorders, and conditions associated with or responsive to the modulation of potassium channels.

Potassium channels (K⁺ channels) are present in nearly all cells and play a crucial role in a wide variety of cellular regulation processes due to modulation of the membrane potential. K⁺ channels can be regulated by changes in membrane voltage, internal Ca²⁺ concentration, phosphorylation, and multiple other cellular mechanisms (Hille, B., Ionic channels in excitable membranes, 2^{nd} ed., *Sinauer Assc.* (1992)). The family of potassium channels can be divided into several subfamilies, one being the group of Ca²⁺-activated K⁺ channels. The potassium channel BK belongs to this subfamily of Ca²⁺- activated K⁺ channels (K_{ca}) and shows a large single channel conductance of ~150pS. The BK channel (or MaxiK), encoded by the *Slo* gene, is mainly regulated by the internal Ca²⁺ concentration and membrane voltage as well as β-subunit modulation, phosphorylation states, and other cellular mechanisms (Nelson M.T. et al., *Science* 270, 633-637 (1995); Levitan, I.B., *Annu. Rev. Physiol.*, 56, 193-212 (1994) ; Vergara *et al.*, *Curr. Opin. Neurobiol.*, 8, 321-329 (1998); McManus, O.B., *Neuron*, 14, 645-650 (1995)). Large conductance, Ca²⁺-activated BK channels are ubiquitously expressed, except in myocardial tissue, and play a key role, e.g. in smooth muscle tone, neuron firing, and cell secretion (Toro, L. *et al.*, From ion channels to cell to cell conversations, *Plenum Press,* NY 47-65, (1997); Fox, A.J. *et al.*, *J. Clin. Invest.,* 99, 513-519 (1997); Nelson M.T. *et al.*, *Science* 270, 633-637 (1995); Lingle C:J. *et al*, *Ion channels,* 4, 4, 261-301 (1996)). The opening of BK channels leads to a shift of the membrane potential towards the potassium reversal potential causing hyperpolarization of the cell. Due to its large single channel conductance the opening of only few BK channels can produce a significant leftward shift of the membrane potential due to the increased K⁺ conductance. Such mechanisms are important for example in smooth muscle cells, where hyperpolarization caused by BK channel opening leads to a relaxation and therefore a reduced vascular tone, or in neuronal tissue, where BK channel opening counteracts depolarisation and can limit the hyperactivating and/or damaging Ca²⁺ entry under different disease conditions. Inhibition of BK channels can maintain or lead to a more depolarized membrane potential of the cell and therefore maintain or prolong cellular processes depending on cellular depolarization.

Other members of the subfamily of Ca²⁺-activated K⁺ channels (K_{Ca}) are SK_{Ca} (SK_{Ca}-1,2,3) and IK_{ca} channels, with small or intermediate conductances, respectively. SK_{ca} and IK_{ca} channels do not show any voltage dependence like the BK channel described above. SK_{ca} channels are expressed in different neuronal tissues, in skeletal muscles, gland cells, liver cells, lymphocytes, and other peripheral cells. SK_{ca} channels are important in mechanisms, where a specific regulation of the cellular membrane potential is required for the normal function of cells, e.g. the after-hyperpolarization in neuronal tissues influencing the firing pattern of neurons. IK_{ca} channels are expressed, e.g. in endothel cell, red blood cells, and lymphocytes. These channels are also responsible for a tightly regulated membrane potential to guarantee a specific cellular function, e.g. the activation processes of T-lymphocytes. Other K⁺ channels that are important for a specific regulation of the membrane potential are K_{ATP} channels. These K⁺ channels belong to the subfamily of channels with 2 transmembranal segments and are inhibited by intracellular ATP. These channels are expressed, e.g. in insulin secreting cells or in vascular muscles, where they have an important role in regulating vascular tone (for review see Coghlan *et al.*, *J. Med. Chem.*, 44, 1627-1653 (2001).

In general, modulation of K⁺ channels by agonistic or antagonistic compounds can influence the membrane potential of K⁺-expressing cells, enabling a specific modulation of cells and/or tissues that might be useful in the treatment of diseases linked to membrane potential or conductance dependent cellular functions.

Several natural and synthetic molecules with the ability to modulate K⁺ channels have been identified in the past. Examples of such compounds are the avena pyrone with BK channel opening activity (WO 93/08800), triaminobenzene analogues were reported to show K⁺ channel opening activity (US 5,200,422), the aryl-pyrrole NS-8 has been disclosed to act as a K⁺ channel opener useful in the treatment of bladder dysfunction (Tanaka, et al., *J. Urol.* 159, 21 (1998)), indole-3-carboxylic acid esters have been shown to exert BK opening activity (Hu et al., *Drug.Dev.Res.* 41, 10 (1997)), benzimidazole derivatives with K_{ATP} and BK opening activity (US 5,475,015), novel compounds (eg. NS004) with K⁺ channel opening activity by Neurosearch (WO 00/69838; WO 00/34248) and 3-substituted oxoindole derivatives with BK-channel opening activity for neuronal protection, especially after ischemic stroke (US 5,602,169).
Isoquinoline-3-carboxylic acid derivatives have been proposed in WO 02/059095 for the treatment of diabetes and sexual dysfunction, 3,4-dihydro-isoquinoline derivatives (WO 01/87844) for the treatment of cancer, autoimmune and infection and 1,2,3,4-tetrahydroisoquinolin-6-ol derivatives are described im WO 02/46164 as medicament for the treatment or prophylaxis of depressive disorders and prostate cancer.
EP 1113007, WO 96/38471 and WO 96/34870 discloses isoquinoline derivatives that are useful in the treatment of endothelium dysfunction, ischemic, diabetes, hypoglycemics, obesity, AIDS, cancer, immunodepressive and other diseases.
Tetrahydroisoquinoline amide derivatives (JP 5339240) show antagonistic action on tachykinin and are useful for the treatment of asthma and cronic bronchitis.
The quinolines in WO 99/42456 are useful for sexual dysfunction, depression, celebra ischemia.
2-acetyl-1,2,3,4-tetrahydro-6,8-dimethoxy-1-(p-methoxybenzyl)-isoquinoline was described in Yakugaku Zasshi (1963), 83, 288-92.

In general, the present invention provides compounds useful for the treatment or alleviation of diseases, disorders, and conditions associated with potassium channels.

The present invention therefore refers to compounds of the general Formula (I) or a salt, or a physiologically functional derivative, or a prodrug thereof, wherein 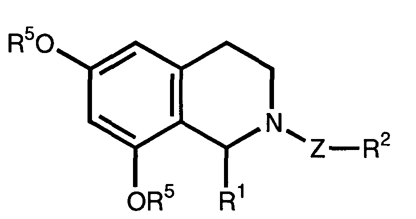 wherein
- Z: is carbonyl or sulfonyl;
- R¹: is alkyl, alkenyl, alkynyl, aryl, H, halogen, haloalkyl, haloalkoxy, hydroxyalkyl, cycloalkyl or heteroaryl;
- R²: is -CH₂-SO₂-alkyl, -CH₂-SO₂-cycloalkyl, -CH₂-SO₂-aryl, -CH₂-SO₂-heteroaryl, aryl, haloalkyl, haloalkoxy, hydroxyalkyl, cycloalkyl, heteroaryl or alkyl, alkenyl, alkynyl;
- R³: is H, alkyl, alkenyl, alkynyl, cycloalkyl, -CO₂R⁴, -CONHR⁴, -CR⁴O, -SO₂R⁴, -NR⁴-CO-R⁴, alkoxy, alkylthio, -OH, -SH, -O-aryl, -O-cycloalkyl, -S-aryl, -S-cycloalkyl, hydroxyalkyl, halogen, -haloalkyl, haloalkoxy, CN, NO₂, hydroxyalkylamine, aminoalkyl, alkylamine, aryl or heteroaryl;
- R⁴: is H, halogen, alkyl, alkenyl, alkynyl, cycloalkyl, alkoxy, alkylthio, -O-aryl, -O-cycloalkyl, OH, SH, -S-aryl, -S-cycloalkyl, hydroxyalkyl, haloalkyl, haloalkoxy, hydroxyalkyl-amine, aminoalkyl, alkylamine, aryl or heteroaryl;
- R⁵: is independently alkyl, alkenyl, alkynyl;
wherein
an alkyl group, if not stated otherwise, denotes a linear or branched C₁-C₁₂-alkyl, which can optionally be substituted by one or more substituents R³, wherein R³ being as defined above; an alkenyl group, if not stated otherwise, denotes a linear or branched C₁-C₁₂-alkenyl, which can optionally be substituted by one or more substituents R³, wherein R³ being as defined above;
an alkynyl group, if not stated otherwise, denotes or a linear or branched C₁-C₁₂-alkynyl group, which can optionally be substituted by one or more substituents R³, wherein R³ being as defined above;
a cycloalkyl group denotes a non-aromatic ring system containing three to eight carbon atoms, wherein one or more of the carbon atoms in the ring can be substituted by a group X, wherein X is selected from the group consisting of N, S, O, SO, SO₂, NR⁴ and CO; the cycloalkyl group can optionally be substituted by one or more substituents R³, wherein R³ being as defined above;
an alkoxy group denotes an O-alkyl group, the alkyl group being as defined above;
an haloalkyl group denotes an alkyl group which is substituted by one to five halogen atoms, the alkyl group being as defined above;
a hydroxyalkyl group denotes an HO-alkyl group, the alkyl group being as defined above;
an haloalkyloxy group denotes an alkoxy group which is substituted by one to five halogen atoms, the alkyl group being as defined above;
a hydroxyalkylamino group denotes an (HO-alkyl)₂-N- group or HO-alkyl-NH- group, the alkyl group being as defined above;
a halogen group is chlorine, bromine, fluorine or iodine;
an aryl group preferably denotes an aromatic group having five to fifteen carbon atoms, which can optionally be substituted by one or more substituents R³, wherein R³ being as defined above;
a heteroaryl group denotes a 5- or 6-membered heterocyclic group which contains at least one heteroatom like O, N, S. This heterocyclic group can be fused to another ring. This heterocyclic group can optionally be substituted by one or more substituents R³, wherein R³ being as defined above;
in the CH₂-SO₂-alkyl group, the alkyl group is being as defined above;
in the CH₂-SO₂-cycloalkyl group, the cycloalkyl group is being as defined above;
in the CH₂-SO₂-aryl group, the aryl group is being as defined above;
in the CH₂-SO₂-heteroary group, the heteroaryl group is being as defined above;
with the provisio that 2-acetyl-1,2,3,4-tetrahydro-6,8-dimethoxy-1-(p-methoxybenzyl)-isoquinoline is excluded.

The present invention therefore refers also to compounds of the general Formula (I) or a salt, or a physiologically functional derivative, or a prodrug thereof, wherein 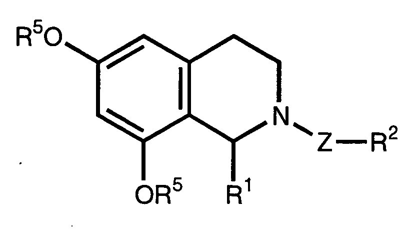 wherein
- Z: is carbonyl or sulfonyl;
- R¹: is alkyl, alkenyl, alkynyl, aryl, H, halogen, haloalkyl, haloalkoxy, hydroxyalkyl, cycloalkyl or heteroaryl;
- R²: is -CH₂-SO₂-alkyl, -CH₂-SO₂-cycloalkyl, -CH₂-SO₂-aryl, -CH₂-SO₂-heteroaryl, aryl, haloalkyl, haloalkoxy, hydroxyalkyl, cycloalkyl, heteroaryl or a linear or branched C₂-C₆-alkyl group, which can optionally be substituted by one or more substituents R³;
- R³: is H, alkyl, alkenyl, alkynyl, cycloalkyl, -CO₂R⁴, -CONHR⁴, -CR⁴O, -SO₂R⁴, -NR⁴-CO-R⁴, alkoxy, alkylthio, -OH, -SH, -O-aryl, -O-cycloalkyl, -S-aryl, -S-cycloalkyl, hydroxyalkyl, halogen, haloalkyl, haloalkoxy, CN, NO₂, hydroxyalkylamine, aminoalkyl, alkylamine, aryl or heteroaryl;
- R⁴: is H, halogen, alkyl, alkenyl, alkynyl, cycloalkyl, alkoxy, alkylthio, -O-aryl, -O-cycloalkyl, OH, SH, -S-aryl, -S-cycloalkyl, hydroxyalkyl, haloalkyl, haloalkoxy, hydroxyalkyl-amine, aminoalkyl, alkylamine, aryl or heteroaryl;
- R⁵: is independently alkyl, alkenyl, alkynyl;
wherein
a C₂-C₆-alkyl group denotes a linear or branched C₂-C₆-alkyl, which can optionally be substituted by one or more substituents R³, wherein R³ being as defined above;
an alkyl group, if not stated otherwise, denotes a linear or branched C₁-C₁₂-alkyl, which can optionally be substituted by one or more substituents R³, wherein R³ being as defined above; an alkenyl group, if not stated otherwise, denotes a linear or branched C₁-C₁₂-alkenyl, which can optionally be substituted by one or more substituents R³, wherein R³ being as defined above;
an alkynyl group, if not stated otherwise, denotes or a linear or branched C₁-C₁₂-alkynyl group, which can optionally be substituted by one or more substituents R³, wherein R³ being as defined above;
the C₁-C₁₂-alkyl, C₁-C₁₂-alkenyl and C₁-C₁₂-alkynyl residue may include but not limited to the following groups -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -CH₂-CH(CH₃)-CH₃, -C(CH₃)₃, -C₂H₄-CH=CH₂, -CH=CH-C₂H₅, -CH=C(CH₃)₂, -CH₂-CH=CH-CH₃, -CH₂-C(CH₃)=CH₂, -C(CH₃)=CH-CH₃, -C(CH₃)-CH=CH₂, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₅H₁₁, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=C(CH₃)₂, -C(CH₃)=C(CH₃)₂, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C(CH₃)=CH-CH=CH₂, -CH=C(CH₃)-CH=CH₂, -CH=CH-C(CH₃)=CH₂, -C₃H₆-C≡CH, -C≡C-C₃H₇, -C₂H₄-C≡C-CH₃, -CH₂-C≡C-C₂H₅, -CH₂-C≡C-C≡CH, -C≡C-C≡C-CH₃, -C≡C-CH₂-C≡CH, -CH₂-C≡C-CH≡CH₂, -CH₂-CH=CH-C≡CH, -C≡C-CH=CH-CH₃, -CH=CH-C≡C-CH₃, -C≡C-CH₂-CH=CH₂, -CH=CH-CH₂-C≡CH, -C(CH₃)=CH-C≡CH, -CH=C(CH₃)-C≡CH, -C≡C-C(CH₃)=CH₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CN₂-C(CH₃)=C(CH₃)₂, -C₂H₄-CH=C(CH₃)₂, -CH₂-CH=CH-CH(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅;-C₇H₁₅, -C₃H₆-C(CH₃)₃, -C₄H₈-CH(CH₃)₂, -C₃H₆-CH(CH₃)-C₂H₅, -C₂H₄-C(CH₃)₂-C₂H₅, -C₂H₄-CH(CH₃)-C₃H₇, -CH₂-C(CH₃)₂-C₃H₇, -CH₂-CH(CH₃)-C₄H₉,-CH(CH₃)-C₅H₁₁, -C(CH₃)₂-C(CH₃)₂-CH₃, -C(CH₃)₂-CH₂-CH(CH₃)₂, -CH(CH₃)-C₂H₄-CH(CH₃)₂, -CH₂-CH(CH₃)-CH₂-CH(CH₃)₂, -CH₂-C(CH₃)₂-CH(CH₃)₂, -CH(CH₃)-CH₂-C(CH₃)₃, -CH₂-CH(CH₃)-C(CH₃)₃, -CH=CH-C₅H₁₁, -CH₂-CH=CH-C₄H₉, -C₂H₄-CH=CH-C₃H₇, -C₃H₆-CH=CH-C₂H₅, -C₄H₈-CH=CH-CH₃, -C₅H₁₀-CH=CH₂, -CH=CH-CH₂-C(CH₃)₃, -CH=CH-C(CH₃)₂-C₂H₅, -C(CH₃)₂-CH(CH₃)-CH=CH₂ -C≡C-C₅H₁₁, -CH₂-C≡C-C₄H₉, -C₂H₄-C≡C-C₃H₇, -C₃H₆-C≡C-C₂H₅, -C₄H₈-C≡C-CH₃, -C₅H₁₀-C≡CH, -C≡C-CH₂-C(CH₃)₃, -C≡C-C(CH₃)₂-C₂H₅, -C(CH₃)₂-CH(CH₃)-C≡CH, -C₈H₁₇, -C₄H₈-C(CH₃)₃, -C₅H₁₀-CH(CH₃)₂, -C₄H₈-CH(CH₃)-C₂H₅, -C₃H₆-C(CH₃)₂-C₂H₅, -C₃H₆-CH(CH₃)-C₃H₇, -C₂H₄-C(CH₃)₂-C₃H₇, -C₂H₄-CH(CH₃)-C₄H₉, -CH₂-C(CH₃)₂-C₄H₉, -CH₂-CH(CH₃)-C₅H₁₁, -C(CH₃)₂-C₅H₁₁, -CH(CH₃)-C₆H₁₃, -C₄H₈-C(CH₃)₂-CH₃, -CH₂-C(CH₃)₂-C(CH₃)₃, -C(CH₃)₂-CH₂-C(CH₃)₃, -CH(CH₃)-CH(CH₃)-C(CH₃)₃, -CH₂-C(CH₃)₂-CH₂-CH(CH₃)₂, -C(CH₃)₂-C₂H₄-CH(CH₃)₂, -C₂H₄-C(CH₃)₂-CH(CH₃)₂, -CH(CH₃)-C₃H₆-CH(CH₃)₂, -C₂H₄-CH(CH₃)-CH₂-CH(CH₃)₂, -CH(CH₃)-C₂H₄-C(CH₃)₃, -CH₂-CH(CH₃)-CH₂-C(CH₃)₃, -C₂H₄-CH(CH₃)-C(CH₃)₃, -CH=CH-C₆H₁₂, -CH₂-CH=CH-C₅H₁₁, -C₂H₄-CH=CH-C₄H₉, -C₃H₆-CH=CH-C₃H₇, -C₄H₈-CH=CH-C₂H₅, -C₅H₁₀-CH=CH-CH₃, -C₆H₁₂-CH=CH₂, -CH=CH-C₂H₄-C(CH₃)₃, -CH=CH-C(CH₃)₂-C₃H)₂, -C(CH₃)₂-CH(CH₃)-CH₂-CH=CH₂, -C≡C-C₆H₁₃, -CH₂-C≡C-C₅H₁₁, -C₂H₄-C≡C-C₄H₉, -C₃H₆-C≡C-C₃H₇, -C₄H₈-C≡C-C₂H₅, -C₅H₁₀-C≡C-CH₃, -C₆H₁₂-C≡CH, -C≡C-C₂H₄-C(CH₃)₃, -C≡C-C(CH₃)₂-C₃H₇, -C(CH₃)₂-CH(CH₃)-CH₂-C≡CH, -C(CH₃)₂-CH₂-CH(CH₃)-C≡CH, -C₉H₁₉, -C₅H₁₀-C(CH₃)₃, -C₆H₁₂-CH(CH₃)₂, -C₅H₁₀-CH(CH₃)-C₂H₅, -C₄H₈-C(CH₃)₂-C₂H₅, -C₄H₈-CH(CH₃)-C₃H₇, -C₃H₆-C(CH₃)₂-C₃H₇, -C₃H₆-CH(CH₃)-C₄H₉, -C₂H₄-C(CH₃)₂-C₄H₉, -C₂H₄-CH(CH₃)-C₅H₁₁, -CH₂-C(CH₃)₂-C₅H₁₁, -CH₂-CH(CH₃)-C₆H₁₃, -C(CH₃)₂-C₆H₁₃, -CH(CH₃)-C₇H₁₅, -C₂H₄-C(CH₃)₂-C(CH₃)₃, -CH₂-C(CH₃)₂-CH₂-C(CH₃)₃, -C(CH₃)₂-C₂H₄-C(CH₃)₃, -CH₂-CH(CH₃)-CH(CH₃)-C(CH₃)₃, -CH(CH₃)-CH₂-CH(CH₃)-C(CH₃)₃, -CH(CH₃)-CH(CH₃)-CH₂-C(CH₃)₃, -C₂H₄-C(CH₃)₂-CH₂-CH(CH₃)₂, -CH₂-C(CH₃)₂-C₂H₄-CH(CH₃)₂, -C(CH₃)₂-C₃H₆-CH(CH₃)₂, -C₃H₆-C(CH₃)₂-CH(CH₃)₂, -CH(CH₃)-C₄H₈-CH(CH₃)₂, -C₃H₆-CH(CH₃)-CH₂-CH(CH₃)₂, -C₂H₄-CH(CH₃)-C₂H₄-CH(CH₃)₂, -CH₂-CH(CH₃)-C₃H₆-CH(CH₃)₂, -C(CH₃)₂-C(CH₃)₂-CH(CH₃)₂, -C(CH₃)₂-CH(CH₃)-C(CH₃)₃, -CH(CH₃)-C₃H₆-C(CH₃)₃, -C₂H₄-CH(CH₃)-CH₂-C(CH₃)₃, -CH₂-CH(CH₃)-C₂H₄-C(CH₃)₃, -C₃H₆-CH(CH₃)-C(CH₃)₃, -C(CH₃)₂-CH(CH₃)-C(CH₃)₃, -CH=CH-C₇H₁₅, -CH₂-CH=CH-C₆H₁₃, -C₂H₄-CH=CH-C₅H₁₁, -C₃H₆-CH=CH-C₄H₉, -C₄H₈-CH=CH-C₃H₇, -C₅H₁₀-CH=CH-C₂H₅, -C₆H₁₀-CH=CH-CH₃, -C₇H₁₄-CH=CH₂, -CH=CH-C₃H₆-C(CH₃)₃, -CH=CH-C(CH₃)₂-C₄H₉, -C(CH₃)₂-CH(CH₃)-C₂H₄-CH=CH₂, -C(CH₃)₂-C(CH₃)₂-CH₂-CH=CH₂, -C≡C-C₇H₁₅, -CH₂-C≡C-C₆H₁₃, -C₂H₄-C≡C-C₅H₁₁, -C₃H₆-C≡C-C₄H₉, -C₄H₈-C≡C-C₃H₇, -C₅H₁₀-C≡C-C₂H₅, -C₆H₁₂-C≡C-CH₃, -C₇H₁₄-C≡CH, -C≡C-C₃H₆-C(CH₃)₃, -C≡C-C(CH₃)₂-C₄H₉, -C≡C-C(CH₃)₂-C(CH₃)₂-CH₃, -C(CH₃)₂-CH(CH₃)-C₂H₄-C≡CH, -C(CH₃)₂-C₂H₄-CH(CH₃)-C≡CH, -C≡C-C(CH₃)₂-C(CH₃)₃, -C₁₀H₂₁, -C₆H₁₂-C(CH₃)₃, -C₇H₁₄-CH(CH₃)₂, -C₆H₁₂-CH(CH₃)-C₂H₅, -C₅H₁₀-C(CH₃)₂C₂H₅, -C₅H₁₀-CH(CH₃)-C₃H₇, -C₄H₈-C(CH₃)₂-C₃H₇, -C₄H₈-CH(CH₃)-C₄H₉, -C₃H₆-C(CH₃)₂-C₄H₉, -C₃H₆-CH(CH₃)-C₅H₁₁, -C₂H₄-C(CH₃)₂-C₅H₁₁, -C₂H₄-CH(CH₃)-C₆H₁₃, -CH₂-C(CH₃)₂-C₆H₁₃, -CH₂-CH(CH₃)-C₇H₁₅, -C(CH₃)₂-C₇H₁₅, -CH(CH₃)-C₈H₁₇, -C₃H₆-C(CH₃)₂-C(CH₃)₃, -C₂H₄-C(CH₃)₂-CH₂-C(CH₃)₃, -C(CH₃)₂-C₃H₆-C(CH₃)₃, -C₂H₄-CH(CH₃)-CH(CH₃)-C(CH₃)₃, -CH(CH₃)-C₂H₄-CH(CH₃)-C(CH₃)₃, -CH(CH₃)-CH(CH₃)-C₂H₄-C(CH₃)₃, -C(CH₃)₂-C(CH₃)₂-C(CH₃)₃, -C₃H₆-C(CH₃)₂-CH₂-CH(CH₃)₂, -CH₂-C(CH₃)₂-C₃H₆-CH(CH₃)₂, -C(CH₃)₂-C₄H₈-CH(CH₃)₂, -C₄H₈-C(CH₃)₂-CH(CH₃)₂, -CH(CH₃)-C₅H₁₀-CH(CH₃)₂, -C₄H₈-CH(CH₃)-CH₂-CH(CH₃)₂, -C₂H₄-CH(CH₃)-C₃H₆-CH(CH₃)₂, -CH₂-CH(CH₃)-C₄H₈-CH(CH₃)₂, -CH(CH₃)-C₄H₈-C(CH₃)₃, -C₃H₆-CH(CH₃)-CH₂-C(CH₃)₃, -CH₂-CH(CH₃)-C₃H₆-C(CH₃)₃, -C₄H₈-CH(CH₃)-C(CH₃)₃, -CH=CH-C₈H₁₇, -CH₂-CH=CH-C₇H₁₅, -C₂H₄-CH=CH-C₆H₁₃, -C₃H₆-CH=CH-C₅H₁₁, -C₄H₈=CH=CH-C₄H₉, -C₅H₁₀-CH=CH-C₃H₇, -C₆H₁₂-CH=CH-C₂H₅, -C₇H₁₄-CH=CH-CH₃, -C₈H₁₆-CH=CH₂, -CH=CH-C₄H₈-C(CH₃)₃, -CH=CH-C(CH₃)₂-C₅H₁₁, -C(CH₃)₂-CH(CH₃)-C₃H₆-CH=CH₂, -C(CH₃)₂-C(CH₃)₂-C₂H₄-CH=CH₂, -C(CH₃)₂-C(CH₃)₂-CH(CH₃)-CH=CH₂, -C≡C-C₈H₁₇, -CH₂-C≡C-C₇H₁₅, -C₂H₄-C≡C-C₆H₁₃, -C₃H₆-C≡C-C₅H₁₁, -C₄H₈-C≡C-C₄H₉, -C₅H₁₀-C≡C-C₃H₇, -C₆H₁₂-C≡C-C₂H₅, -C₇H₁₄-C≡C-CH₃, -C₈H₁₆-C≡CH, -C≡C-C₄H₈-C(CH₃)₃, -C≡C-C(CH₃)₂-C₅H₁₁, -C≡C-C(CH₃)₂-C(CH₃)₂-C₂H₅, -C(CH₃)₂-CH(CH₃)-C₃H₆-C≡CH, -C(CH₃)₂-C₃H₆-CH(CH₃)-C≡CH, -C₁₁H₂₃, -C₇H₁₄-C(CH₃)₃, -C₈H₁₆-CH(CH₃)₂, -C₇H₁₄-CH(CH₃)-C₂H₅, -C₆H₁₂-C(CH₃)₂-C₂H₅, -C₆H₁₂-CH(CH₃)-C₃H₇, -C₅H₁₀-C(CH₃)₂-C₃H₇, -C₅H₁₀-CH(CH₃)-C₄H₉, -C₄H₈-C(CH₃)₂-C₄H₉, -C₄H₈-CH(CH₃)-C₅H₁₁, -C₃H₆-C(CH₃)₂-C₅H₁₁, -C₃H₆-CH(CH₃)-C₆H₁₃, -C₂H₄-C(CH₃)₂-C₆H₁₃, -C₂H₄-CH(CH₃)-C₇H₁₅, -CH₂-C(CH₃)₂-C₇H₁₅, -CH₂-CH(CH₃)-C₈H₁₇, -C(CH₃)₂-C₈H₁₇, -CH(CH₃)-C₉H₁₉, -C₄H₈-C(CH₃)₂-C(CH₃)₃, -C₂H₄-C(CH₃)₂-C₂H₄-C(CH₃)₃, -C(CH₃)₂-C₄H₈-C(CH₃)₃, -C₃H₆-CH(CH₃)-CH(CH₃)-C(CH₃)₃, -CH(CH₃)-C₃H₆-CH(CH₃)-C(CH₃)₃, -CH(CH₃)-CH(CH₃)-C₃H₆-C(CH₃)₃, -C₃H₆-C(CH₃)₂-C₂H₄-CH(CH₃)₂, -C₂H₄-C(CH₃)₂-C₃H₆-CH(CH₃)₂, -C(CH₃)₂-C₅H₁₀-CH(CH₃)₂, -C₅H₁₀-C(CH₃)₂-CH(CH₃)₂, -C(CH₃)₂-C(CH₃)₂-CH(CH₃)-CH(CH₃)₂, -CH (CH₃)-C₆H₁₂-CH (CH₃)₂, -C₄H₈-CH(CH₃)-C₂H₄-CH(CH₃)₂, -C₃H₆-CH(CH₃)-C₃H₆-CH(CH₃)₂, -C₂H₄-CH(CH₃)-C₄H₈-CH(CH₃)₂, -CH(CH₃)-C₅H₁₀-C(CH₃)₃, -C₃H₆-CH(CH₃)-C₂H₄-C(CH₃)₃, -C₂H₄-CH(CH₃)-C₃H₆-C(CH₃)₃, -C₅H₁₀-CH(CH₃)-C(CH₃)₃, -CH(CH₃)-C(CH₃)₂-CH(CH₃)-C(CH₃)₃, -CH=CH-C₉H₁₉, -C₂H₄-CH=CH-C₇H₁₅, -C₃H₆-CH=CH-C₆H₁₃, -C₄H₈-CH=CH-C₅H₁₁, -C₅H₁₀-CH=CH-C₄H₉, -C₆H₁₂-CH=CH-C₃H₇, -C₇H₁₄-CH=CH-C₂H₅, -C₈H₁₆-CH=CH-CH₃, -C₉H₁₈-CH=CH, -CH=CH-C₅H₁₀-C(CH₃)₃, -CH=CH-C(CH₃)₂-C₆H₁₃, -C(CH₃)₂-CH(CH₃)-C₄H₈-CH=CH₂, -C(CH₃)₂-C(CH₃)₂-C₃H₆-CH=CH₂, -C(CH₃)₂-C(CH₃)₂-C(CH₃)₂-CH=CH₂, -C≡C-C₉H₁₉, -C₂H₄-C≡C-C₇H₁₅, -C₃H₆-C≡C-C₆H₁₂, -C₄H₈-C≡C-C₅H₁₁, -C₅H₁₀-C≡C-C₄H₉, -C₆H₁₂-C≡C-C₃H₇, -C₇H₄-C≡C-C₂H₅, -C₈H₁₆-C≡C-CH₃, -C₉H₁₈-C≡CH, -C≡C-C₅H₁₀-C(CH₃)₃, -C≡C-C(CH₃)₂-C₆H₁₃, -C≡C-C(CH₃)₂-C( CH₃)₂-C₃H₇, -C(CH₃)₂-CH(CH₃)-C₄H₈-C≡CH, -C(CH₃)₂-C₄H₈-CH(CH₃)-C≡CH, -C≡C-CH(CH₃)-C(CH₃)₂-C(CH₃)₃, -C₁₂H₂₅, -C₈H₁₆-C(CH₃)₃, -C₉H₁₈-CH(CH₃)₂, -C₈H₁₆-CH(CH₃)-C₂H₅, -C₇H₁₄-C(CH₃)₂-C₂H₅, -C₇H₁₄-CH(CH₃)-C₃H₇, -C₆H₁₂-C(CH₃)₂-C₃H₇, -C₆H₁₂-CH(CH₃)-C₄H₉, -C₅H₁₀-C(CH₃)₂-C₄H₉, -C₅H₁₀-CH(CH₃)-C₅H₁₁, -C₄H₈-C(CH₃)₂-C₅H₁₁, -C₄H₈-CH(CH₃)-C₆H₁₁, -C₃H₆-C(CH₃)₂-C₆H₁₃, -C₃H₆-CH(CH₃)-C₇H₁₅, C₂H₄-C(CH₃)₂-C₇H₁₅, -C₂H₄-CH(CH₃)-C₈H₁₇, CH₂-C(CH₃)₂-C₈H₁₇, -CH₂-CH(CH₃)-C₉H₁₉, -C(CH₃)₂-C₉H₁₉, -CH(CH₃)-C₁₀H₂₁, -C₅H₁₀-C(CH₃)₂-C(CH₃)₃, -C₃H₆-C(CH₃)₂-C₂H₄-C(CH₃)₃, -C(CH₃)₂-C₅H₁₀-C(CH₃)₃, -C₄H₈-CH(CH₃)-CH(CH₃)-C(CH₃)₃, -CH(CH₃)-C₄H₈-CH(CH₃)-C(CH₃)₃, -CH(CH₃)-CH(CH₃)-C₄H₈-C(CH₃)₃, -C(CH₃)₂-CH(CH₃)-C(CH₃)₂-C(CH₃)₃, -C₄H₈-C(CH₃)₂-C₂H₄-CH(CH₃)₂, -C₃H₆-C(CH₃)₂-C₃H₆-CH(CH₃)₂, -C(CH₃)₂-C₆H₁₂-CH(CH₃)₂, -C₆H₁₂-C(CH₃)₂-CH(CH₃)₂, -C(CH₃)₂-C(CH₃)₂-C(CH₃)₂-CH(CH₃)₂, -CH(CH₃)-C₇H₁₄-CH(CH₃)₂, -C₅H₁₀-CH(CH₃)-C₂H₄-CH(CH₃)₂, -C₄H₈-CH(CH₃)-C₃H₆-CH(CH₃)₂, -C₃H₆-CH(CH₃)-C₄H₈-CH(CH₃)₂, -CH(CH₃)-C₆H₁₂-C(CH₃)₃, -C₄H₈-CH(CH₃)-C₂H₄-C(CH₃)₃, -C₃H₆-CH(CH₃)-C₃H₆-C(CH₃)₃, -C₆H₁₂-CH(CH₃)-C(CH₃)₃, -C(CH₃)₂- C(CH₃)₂-CH(CH₃)-C(CH₃)₃, -CH=CH-C₁₀H₂₁, -C₃H₆-CH=CH-C₇H₁₅, -C₄H₈-CH=CH-C₆H₁₃, -C₅H₁₀-CH=CH-C₅H₁₁, -C₆H₁₂-CH=CH-C₄H₉, -C₇H₁₄-CH=CH-C₃H₇, -C₈H₁₆-CH=CH-C₂H₅, -C₉H₁₈-CH=CH-CH₃, -C₁₀H₂₀-CH=CH, -CH=CH-C₆H₁₂-C(CH₃)₃, -CH=CH-C(CH₃)₂-C₇H₁₄, -C(CH₃)₂-CH(CH₃)-C₅H₁₀-CH=CH₂, -C(CH₃)₂-C(CH₃)₂-C₄H₈-CH=CH₂ -C≡C-C₁₀H₂₁, -C₃H₆-C≡C-C₇H₁₅, -C₄H₈-C≡C-C₆H₁₂, -C₅H₁₀-C≡C-C₅H₁₁, -C₆H₁₂-C≡C-C₄H₉, -C₇H₁₄-C≡C-C₃H₇, -C₈H₁₆-C≡C-C₂H₅, -C₉H₁₈-C≡C-CH₃, -C₁₀H₂₀-C≡CH, -C≡C-C₆H₁₂-C(CH₃)₃, -C≡C-C(CH₃)₂-C₇H₁₅, -C≡C-C(CH₃)₂-C(CH₃)₂-C₄H₉, -C(CH₃)₂-CH(CH₃)-C₅H₁₀-C≡CH, -C(CH₃)₂-C₅H₁₀-CH(CH₃)-C≡CH,
a cycloalkyl group denotes a non-aromatic ring system containing three to eight carbon atoms, preferably four to eight carbon atoms, wherein one or more of the carbon atoms in the ring can be substituted by a group X, wherein X is selected from the group consisting of N, S, O, SO, SO₂, NR⁴ and CO; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅; the cycloalkyl group can optionally be substituted by one or more substituents R³, wherein R³ being as defined above;
an alkoxy group denotes an O-alkyl group, the alkyl group being as defined above; the alkoxy group is preferably a methoxy, ethoxy, isopropoxy, t-butoxy or pentoxy group;
an haloalkyl group denotes an alkyl group which is substituted by one to five halogen atoms, the alkyl group being as defined above; the haloalkyl group is preferably a -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇ or -C₂H₄-C(R¹⁰)₃, wherein R¹⁰, R^{10'}, R^{10"} represent F, Cl, Br or I, preferably F;
a hydroxyalkyl group denotes an HO-alkyl group, the alkyl group being as defined above;
an haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"}, -OC₂(R¹⁰)₅, -OCH₂-C(R¹⁰)₃, -OCH₂-CR¹⁰(R^{10'})₂, -OCH₂-CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F;
a hydroxyalkylamino group denotes an (HO-alkyl)₂-N- group or HO-alkyl-NH- group, the alkyl group being as defined above;
a halogen group is chlorine, bromine, fluorine or iodine;
an aryl group preferably denotes an aromatic group having five to fifteen carbon atoms, which can optionally be substituted by one or more substituents R³, wherein R³ being as defined above; the aryl group is preferably a phenyl group, -CH₂-C₆H₅, -C₂H₄- C₆H₅, -CH=CH-C₆H₅, -C≡C-C₆H₅, -o-C₆H₄-R³, -m-C₆H₄-R³, -p-C₆H₄-R³, -o-CH₂-C₆H₄-R³, -m-CH₂-C₆H₄-R³, -p-CH₂-C₆H₄-R³;
a heteroaryl group denotes a 5- or 6-membered heterocyclic group which contains at least one heteroatom like O, N, S. This heterocyclic group can be fused to another ring. For example, this group can be selected from an oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, 1,2,5-oxadiazol-4-yl, 1,2,5-thiadiazol-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo-[b]-furanyl, benzo[b]thiophenyl, benzimidazolyl, benzothiazolyl, quinazolinyl, quinoxazolinyl, indolizin-yl, isoindolyl, 3H-indolyl, 1H-indazolyl, benzo[1,3]dioxol-3-yl, benzo[1,3]dioxol-4-yl, benzo[1,3]dioxol-5-yl, 4H-quinolizinyl, isoxazol-3-yl, isoxazol-4-yl, isoxazol-5-yl, quinolinyl, tetrahydroquinolinyl, isoquinolinyl, tetrahydroisoquinolinyl, phthalazinyl, 1,8-naphthyridinyl, pteridinyl, carbazolyl, acridinyl, phenazinyl, phenothiazinyl, phenoxazinyl, indenyl, naphthalinyl, fluorenyl, anthracenyl group. This heterocyclic group can optionally be substituted by one or more substituents R³, wherein R³ being as defined above;
in the CH₂-SO₂-alky group, the alkyl group is being as defined above;
in the CH₂-SO₂-cycloalkyl group, the cycloalkyl group is being as defined above;
in the CH₂-SO₂-aryl group, the aryl group is being as defined above;
in the CH₂-SO₂-heteroaryl group, the heteroaryl group is being as defined above;

In addition, the invention provides methods for preparing compounds of Formula (I).

A first method for the synthesis of 6,8-dimethoxy-isoquinolines of Formula (I) comprises the step of reacting 6,8-dimethoxy-isoquinolines of Formula (V) with a chloride of Formula (VI) For example, this reaction is described in *J. Heterocyclic Chem.* **1992**, *29*, 33-49. 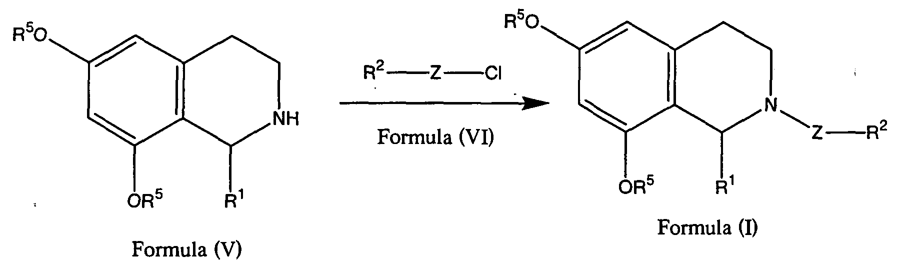 For preparing the 6,8-dimethoxy-isoquinolines of Formula (V) the amide of Formula (IV) is first treated with phosphorous oxychloride then hydrated with sodium borohydride. For example, this reaction is described in *J. Heterocyclic Chem* 1994, 31, 1425-1427 and in *J. Org. Chem.* 1999, *64*, 1115-1120. 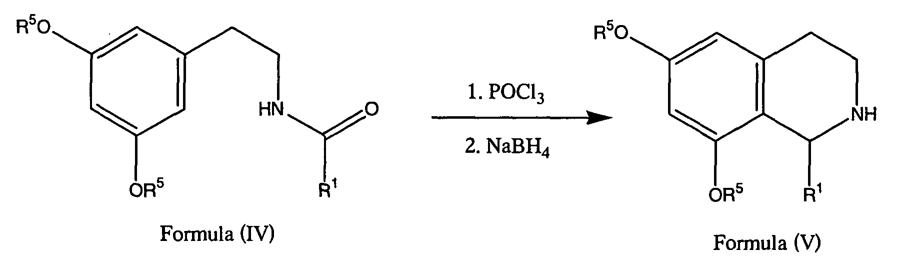 The synthesis of compounds of Formula (IV) comprises the step of reacting the corresponding amine with the corresponding acid chloride (R¹-CO-Cl).

A second method for synthesis of compounds of Formula (I) involves the preparation of tetrahydroisoquinolines utilizing solid-phase combinatorial chemistry. Therefore the aldehyde resin is reacted with the corresponding amine to get resin-bound tetrahydroisoquinolines of Formula (V), which can acylated or sulfonated with a chloride of Formula (VI). 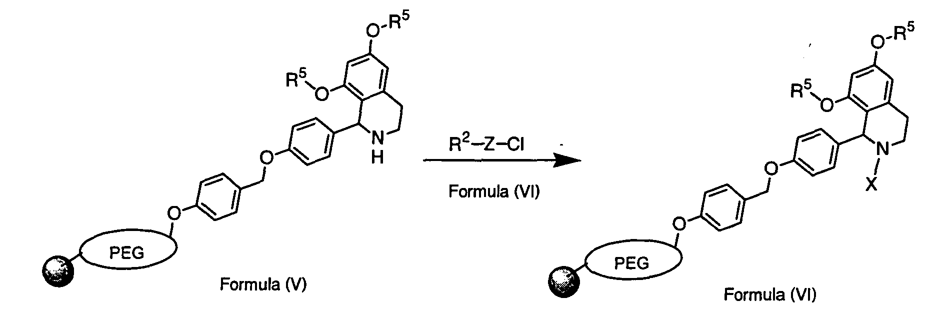 The final step corresponds to the release of the resin from the desired compounds of Formula (I). For example, this method is described in Tetrahedron Letters, 1995, 36, No.50, 9211-9214, Tetrahedron Letters, 1996, 37, No.32, 5633-5636, Tetrahedron Letters, 1996, 37, No.28, 4865-4868, 1996, Tetrahedron Letters, 1995, 36, No.42, 7709-7712, 1995, Synlett, 1996, 1036-1038, and in Cominatorial Chemistry & High Throughput Screening, 2002, 5, 75-81.

In a preferred embodiment of the invention, Z is CO and R¹ is a phenyl group.
In other preferred embodiments, Z is CO and R¹ is a methyl group.
In other preferred embodiments, Z is SO₂ and R¹ is a phenyl group.
In other preferred embodiments, Z is SO₂ and R¹ is a methyl group.
In other preferred embodiments, both R⁵ are methyl groups.
Particularly preferred compounds are those in which at least R¹ or at least R² is a phenyl group, compounds in which R¹ and R² are each a phenyl group being most preferred.
Preferred compounds are those in which R³ is halogen, nitro, tert.-butyl, methyl, OH, OCF₃, CF₃ or hydrogen.

Preferred compounds of the present invention and/or pharmaceutically accetable salts thereof are selected from the group comprising:
(1-Biphenyl-4-yl-6, 8-dimethoxy-3,4-dihydro-1H-isoquinolin-2-yl)-(4-methoxy-phenyl)-methanone, (1-Biphenyl-4-yl-6,8-dimethoxy-3,4-dihydro-1H-isoquinolin-2-yl)-(4-nitrophenyl)-methanone, (1-Biphenyl-4-yl-6,8-dimethoxy-3,4-dihydro-1H-isoquinolin-2-yl)-(3-bromo-phenyl)-methanone, (1-Biphenyl-4-yl-6,8-dimethoxy-3,4-dihydro-1H-isoquinolin-2-yl)-(4-trifluoromethyl-phenyl)-methanone, (1-Biphenyl-4-yl-6,8-dimethoxy-3,4-dihydro-1H-isoquinolin-2-yl)-(2-fluoro-4-trifluoromethyl-phenyl)-methanone, (1-Biphenyl-4-yl-6,8-di-methoxy-3,4-dihydro-1H-isoquinolin-2-yl)-(4-tert-butyl-phenyl)-methanone, (1-Biphenyl-4-yl-6,8-dimethoxy-3,4-dihydro-1H-isoquinolin-2-yl)-(4-trifluoromethoxy-phenyl)-methanone, (1-Biphenyl-4-yl-6,8-dimethoxy-3,4-dihydro-1H-isoquinolin-2-yl)-(3,5-bis-trifluoromethylphenyl)-methanone, [1-(4-Hydroxy-phenyl)-6,8-dimethoxy-3,4-dihydro-1H-isoquinolin-2-yl]-(3-methoxy-phenyl)-methanone, 5-Bromo-1-[1-(4-liydroxy-phenyl)-6,8-dimethoxy-3,4-di-hydro-1H-isoquinolin-2-yl]-pentan-1-one, [1-(4-Hydroxy-phenyl)-6,8-dimethoxy-3,4-di-hydro-1H-isoquinolin-2-yl]-(3-trifluoromethoxy-phenyl)-methanone, 1-[1-(4-Hydroxyphenyl)-6,8-dimethoxy-3,4-dihydro-1H-isoquinolin-2-yl]-3-phenyl-propan-1-one, [1-(4-Hydroxy-phenyl)-6,8-dimethoxy-3,4-dihydro-1H-isoquinolin-2-yl]-(2-trifluoromethoxyphenyl)-methanone, (4-tert-Butyl-phenyl)-[1-(4-hydroxy-phenyl)-6,8-dimethoxy-3,4-dihydro-1H-isoquinolin-2-yl]-methanone, Biphenyl-4-yl-[1-(4-hydroxy-phenyl)-6,8-dimethoxy-3,4-dihydro-1H-isoquinolin-2-yl]-methanone, [1-(4-Hydroxy-phenyl)-6,8-dimethoxy-3,4-dihydro-1H-isoquinolin-2-yl]-quinoxalin-2-yl-methanone, [1-(4-Hydroxy-phenyl)-6,8-dimethoxy-3,4-dihydro-1H-isoquinolin-2-yl]-naphthalen-1-yl-methanone, [1-(4-Hydroxyphenyl)-6,8-dimethoxy-3,4-dihydro-1H-isoquinolin-2-yl]-(3-trifluoromethyl-phenyl)-methanone, Cyclopentyl-[1-(4-hydroxy-phenyl)-6,8-dimethoxy-3,4-dihydro-1H-isoquinolin-2-yl]-methanone, [1-(4-Hydroxy-phenyl)-6,8-dimethoxy-3,4-dihydro-1H-isoquinolin-2-yl]-(4-trifluoromethyl-phenyl)-methanone, 3-Cyclopentyl-1-[1-(4-hydroxy-phenyl)-6,8-dimethoxy-3,4-dihydro-1H-isoquinolin-2-yl]-propan-1-one, [1-(4-Hydroxy-phenyl)-6,8-dimethoxy-3,4-dihydro-1H-isoquinolin-2-yl]-(4-methoxy-phenyl)-methanone, 3-Cyclohexyl-1-[1-(4-hydroxy-phenyl)-6,8-dimethoxy-3,4-dihydro-1H-isoquinolin-2-yl]-propan-1-one, (3-Chloro-benzo[b]thiophen-2-yl)-[1-(4-hydroxy-phenyl)-6,8-dimethoxy-3,4-dihydro-1H-isoquinolin-2-yl]-methanone, [1-(4-Hydroxy-phenyl)-6,8-dimethoxy-3,4-dihydro-1H-iso-quinolin-2-yl]-thiophen-2-yl-methanone, Benzo[1,3]dioxol-5-yl-[1-(4-hydroxy-phenyl)-6,8-dimethoxy-3,4-dihydro-1H-isoquinolin-2-yl]-methanone, [1-(4-Hydroxy-phenyl)-6,8-dimethoxy-3,4-dihydro-1H-isoquinolin-2-yl]-isoxazol-5-yl-methanone, Cyclohexyl-[1-(4-hydroxy-phenyl)-6,8-dimethoxy-3,4-dihydro-1H-isoquinolin-2-yl]-methanone, 1-[6,8-Dimethoxy-1-(3-trifluoromethoxy-phenyl)-3,4-dihydro-1H-isoquinolin-2-yl]-ethanone, [6,8-Dimethoxy-1-(3-trifluoromethoxy-phenyl)-3,4-dihydro-1H-isoquinolin-2-yl]-(4-trifluoromethoxy-phenyl)-methanone, [6,8-Dimethoxy-1-(3-trifluoromethoxy-phenyl)-3,4-dihydro-1H-isoquinolin-2-yl]-(4-methoxy-phenyl)-methanone, N-{4-[6,8-Dimethoxy-1-(3-trifluoromethoxy-phenyl)-3,4-dihydro-1H-isoquinoline-2-carbonyl]-phenyl}-acetamide, (3,5-Bis-trifluoromethoxy-phenyl)-[6,8-dimethoxy-1-(3-trifluoromethoxy-phenyl)-3,4-dihydro-1H-isoquinolin-2-yl]-methanone, 6,8-Dimethoxy-2-(2-nitro-5-trifluoromethyl-benzenesulfonyl)-1-(3-frifluoromethoxy-phenyl)-1,2,3,4-tetrahydro-isoquinoline, 2-(3,5-Bis-trifluoromethylbenzenesulfonyl)-6,8-dimethoxy-1-(3-trifluoromethoxy-phenyl)-1,2,3,4-tetrahydroiso-quinoline, 6,8-Dimethoxy-1-methyl-2-(3-nitro-benzenesulfonyl)-1,2,3,4-tetrahydroisoquinoline, 2-(3,5-Bis-trifluoromethyl-benzenesulfonyl)-6,8-dimethoxy-1-methyl-1,2,3,4-tetra-hydroisoquinoline, (6,8-Dimethoxy-1-methyl-3,4-dihydro-1H-isoquinolin-2-yl)-(3-nitro-phenyl)-methanone, (6,8-Dimethoxy-1-methyl-3,4-dihydro-1H-isoquinolin-2-yl)-(4-trifluoromethoxy-phenyl)-methanone, (6,8-Dimethoxy-1-methyl-3,4-dihydro-1H-isoquinolin-2-yl)-(4-methoxy-phenyl)-methanone,

The compounds of Formula (I) to be used according to the invention can form salts with inorganic or organic acids or bases. Examples of such salts are, for example, alkali metal salts, in particular sodium and potassium salts, or ammonium salts.

In general, the compounds of the present invention will be useful in the treatment of disorders of a living animal body, including a human, due to their potent potassium channel modulating properties.

Therefore, the compounds of the instant invention will be useful in treating disorders of mammals, including humans, where the modulation of the membrane potential or ion conductances is influencing the effects of the disorders. Such disorders include asthma, cystic fibrosis, obstructive pulmonary disease, convulsions, vascular spasms, urinary incontinence, urinary instability, urinary urgency, bladder spasms, ischemia, cerebral ischemia, traumatic brain injury, neurodegeneration, migraine, pain, psychosis, hypertension, epilepsy, memory and attention deficits, functional bowel disorders, erectile dysfunction, female sexual dysfunction, immune suppression, autoimmune disorders, dysfunction of cellular proliferation, diabetes, premature labour, and other disorders associated with or responsive to the modulation of potassium channels.

The invention provides a pharmaceutical formulation comprising a compound of Formula (I) of the invention or a pharmaceutically acceptable salt or derivative thereof, together with one or more pharmaceutically acceptable carriers therefore, and optionally, other therapeutic and/or prophylactic ingredients. The carrier(s) must be 'acceptable' in the sense of being compatible with the other ingredients of the formulation and not deleterious to the recipient thereof.

Pharmaceutical formulations include those suitable for oral, rectal, nasal, topical (including buccal and sub-lingual), vaginal or parenteral (including intramuscular, subcutaneous, intradermal, and intraveneous) administration or in a form suitable for administration by inhalation or insufflation. The compounds of the invention, together with a conventional adjuvant, carrier, or diluent, may thus be placed into the form of pharmaceutical compositions and unit dosages thereof, and in such form may be employed as solids, liquids or in the form of sterile injectable solutions. If a solid carrier is used, the preparation may be tableted, placed in a hard gelatine capsule in powder or pellet form, or in form of a troche or lozenge. The solid carrier may contain conventional excipients such as binding agents, tableting lubricants, fillers, disintegrants, wetting agents and the like. Tablets may be film coated by conventional techniques. If a liquid carrier is employed, the preparation may be in form of a syrup, emulsion, soft gelatine capsule, sterile vehicle for injection, an aqueous or non-aqueous liquid suspension, or may be a dry product for reconstitution with water or other suitable vehicles before use. Liquid preparations may contain conventional additives such as suspending agents, emulsifying agents, wetting agents, non-aqueous vehicle (including edible oils), preservatives, as well as flavouring and /or colouring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. Administration, however, can also be carried out rectally, e.g., in the form of suppositories, or vaginally , e.g. in the form of pessaries, tampons, creams, or percutaneously, e.g., in the form of ointments, creams or tinctures. Administration directly to the nasal cavity by conventional means can be carried out e.g. by pipette, spray or dropper, administration to the respiratory tract may be achieved by means of an aerosol formulation, e.g. where the active ingredient is provided in a pressurized pack with a suitable propellant, or other suitable application mechanisms.

The pharmaceutical compositions are prepared by conventional techniques appropriate to the desired preparation containing appropriate amounts of the active ingredient, that are, the compounds in this invention. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

A suitable dose of compounds or pharmaceutical compositions thereof for a mammal, especially humans, suffering from, or likely to suffer from any condition as described herein is an amount of active ingredient from about 0.1µg/kg to 500mg/kg body weight. For parenteral administration, the dose may be in the range of 0.1µg/kg to 100mg/kg body weight for intravenous administration. The active ingredient will preferably be administered in equal doses from one to four times daily. The compounds of Formula (I) can also be used in the form of a precursor (prodrug) or a suitably modified form that releases the active compound *in vivo.* Normally, the administered dose will be gradually increased until the optimal effective dosage for the treated host is determined. The optimal administered dosage will be determined by a physician or others skilled in the art, depending on the relevant circumstances including the condition to be treated, the choice of compound to be administered, the route of administration, the sex, age, weight, and the specific response of the treated individual in respect to the severity of the individual's symptoms.

### Examples

### Synthesis of compounds of Formula (IV)

The corresponding chloride of Formula (III) (1.2 eq) was added dropwise to a solution of triethylamine (1 eq), 2-(3,5-dimethoxy-phenyl)-ethylamine (1 eq) in 25 ml THF at 0 °C and was stirred 16 h at room temperature. Then water (0.5 ml) was added and the solution was evaporated to dryness. The crude product was purified by preparative thin layer chromatography (Merck, 20 x 20 cm, Silica gel 60 F₂₅₄, 1 mm) using (CH₂Cl₂:MeOH, 100:5) or (petrolether:EtOAc, 8:2) as eluent.

### Synthesis of 6,8-Dimethoxy-isoquinolines of Formula (V)

A mixture of the corresponding compound of Formula (IV) (1 eq) and phosphorous oxychloride (10 eq) in acetonitrile was refluxed under nitrogen for 90 minutes. The reaction mixture was poured into crushed ice, sodified with sodium hydroxide and extracted three times with dichloromethane. The combined extracts were washed, dried over sodium sulfate and concentrated in vacuo. The crude product was purified by preparative thin layer chromatography (Merck, 20 x 20 cm, Silica gel 60 F₂₅₄, 1 mm) using (CH₂Cl₂:MeOH, 100:5) as eluent.
The sodium borohydride (3 eq) was added slowly at 0°C to a stirred solution of the purified product (1 eq) in 10 ml of methanol. After additional stirring for two hours at room temperature, acetic acid was added to reach pH 7.0 and the solution was concentrated in vacuo. The residue was triturated twice with dichloromethane. The combined extracts were washed, dried over sodium sulfate and concentrated in vacuo. The crude product was purified by preparative thin layer chromatography (Merck, 20 x 20 cm, Silica gel 60 F₂₅₄, 1 mm) using (CH₂Cl₂:MeOH, 9:1) as eluent.

### Synthesis of compounds of Formula (I)

The corresponding compounds of Formula (VI) (1.2 eq) was added dropwise to a solution of 6,8-dimethoxy-isoquinoline of Formula (V) (1 eq) in 10 ml of THF at 0 °C and stirred 16 h at room temperature. Then water (0.5 ml) was added and the solution was evaporated to dryness. The crude product was purified by preparative thin layer chromatography (Merck, 20 x 20 cm, Silica gel 60 F₂₅₄, 1 mm) using (CH₂Cl₂:MeOH, 9:1) or (petrolether:EtOAc, 8:2) as eluent.
Table I: Mass was determined by LC/(+)-ESI and LC/(-)-ESI mass spectrometry, the molecular mass, the NMR data (300.13 MHz, abbreviations: s = singulet, d = doublet, t = triplet, m = multiplet) and the Eₘ assay results are shown. Eₘ Assay results are given as the ratio of the compound effect (50µM) compared to the maximal effect of NS004 (25 or 50µM). Ranges are 0-1 = +. >1- 1.5 = ++. >1.5 = +++.

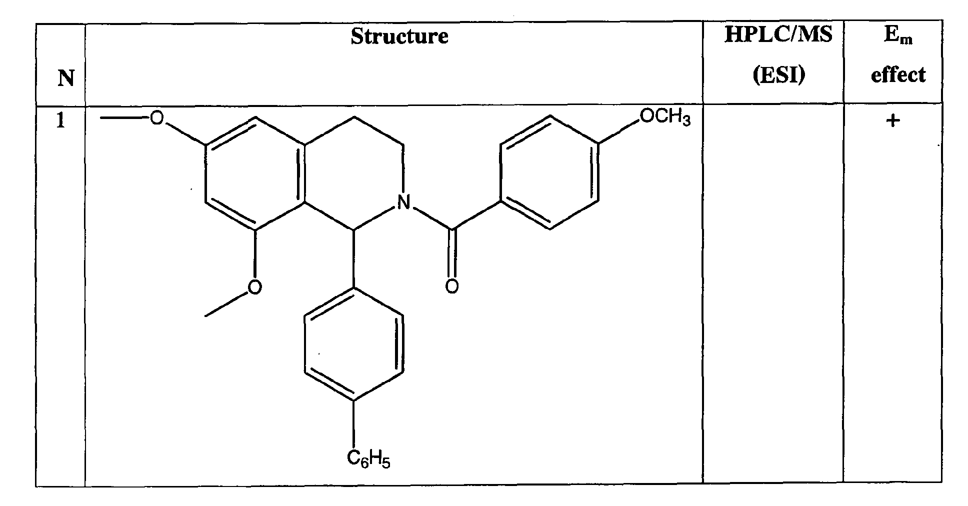

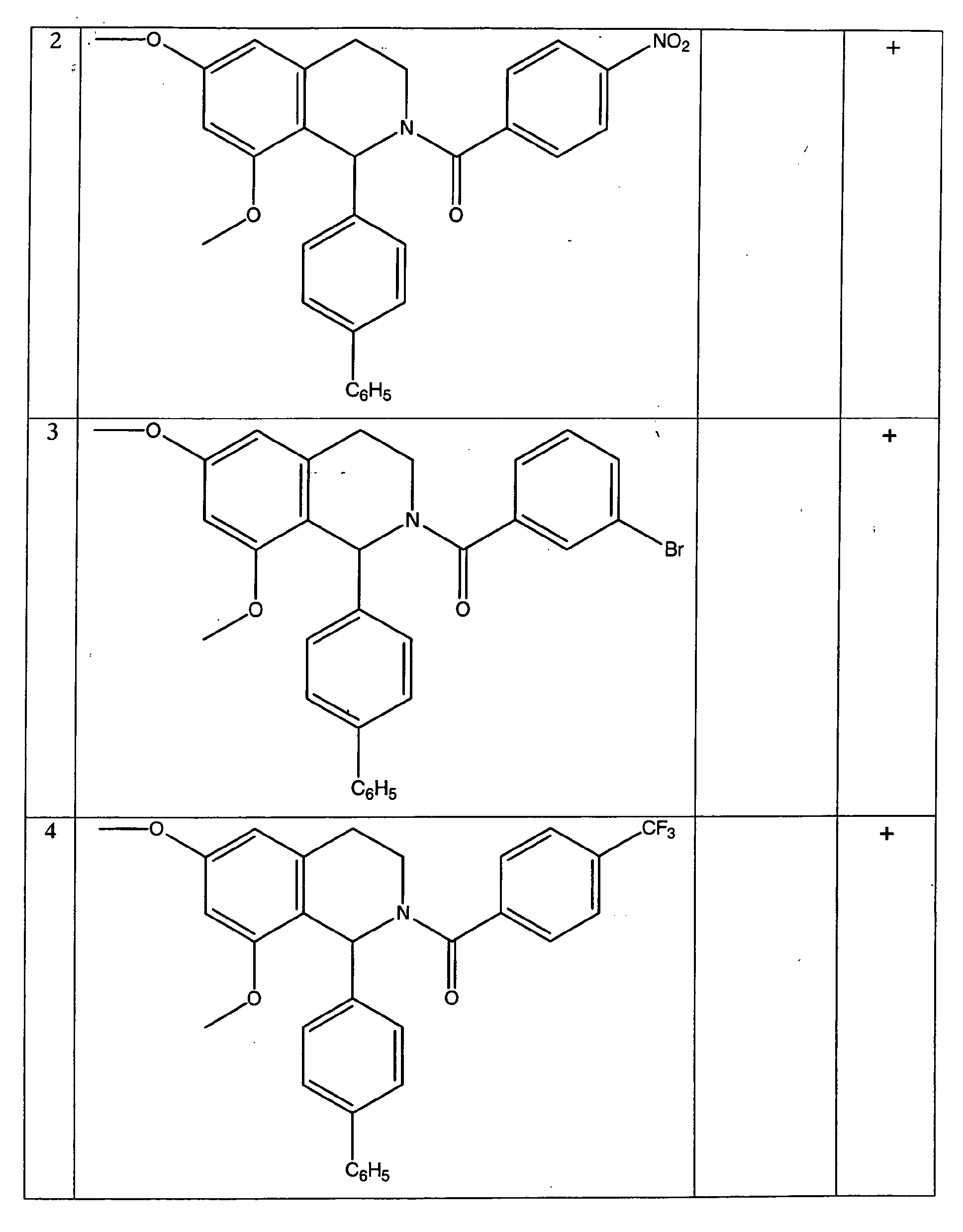

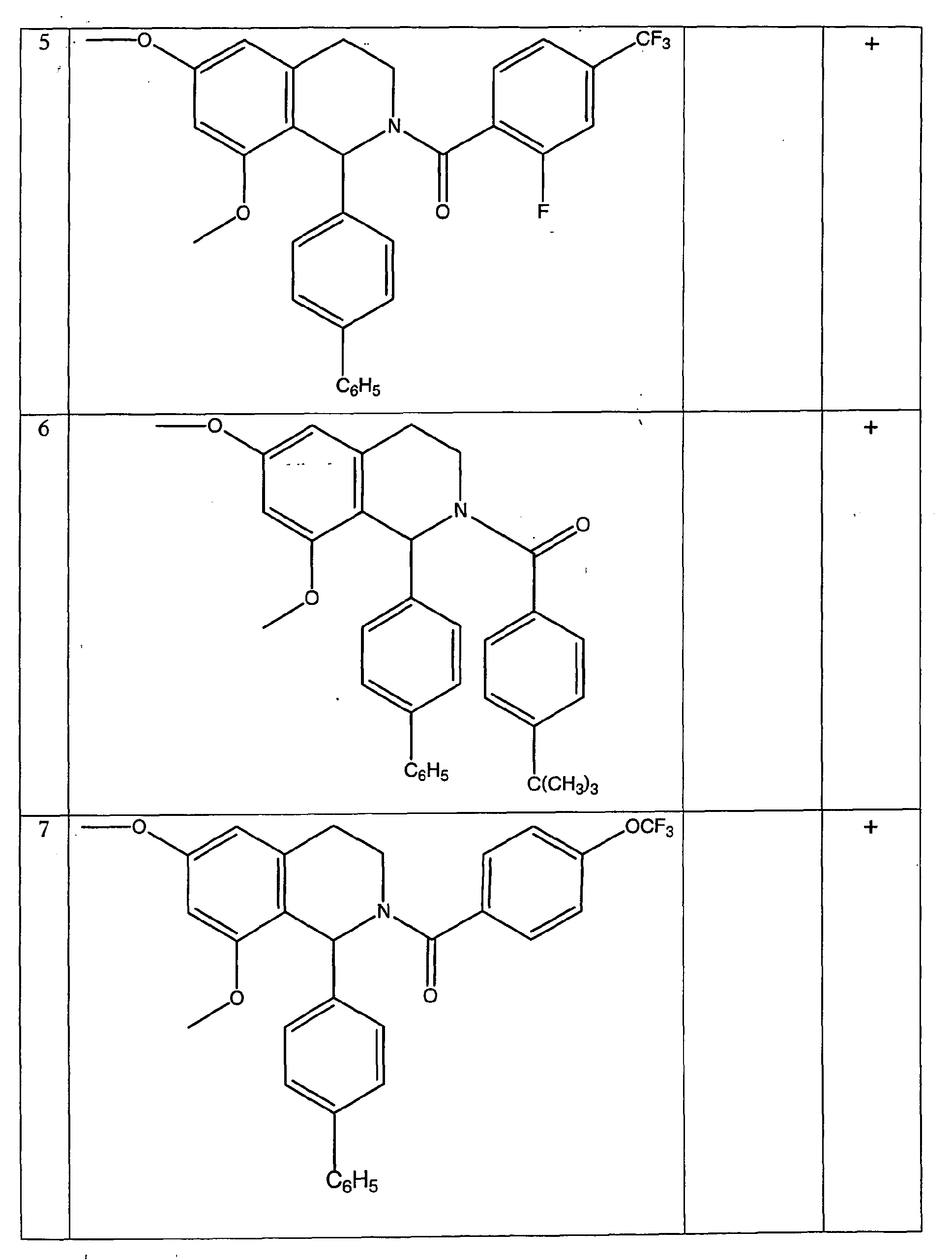

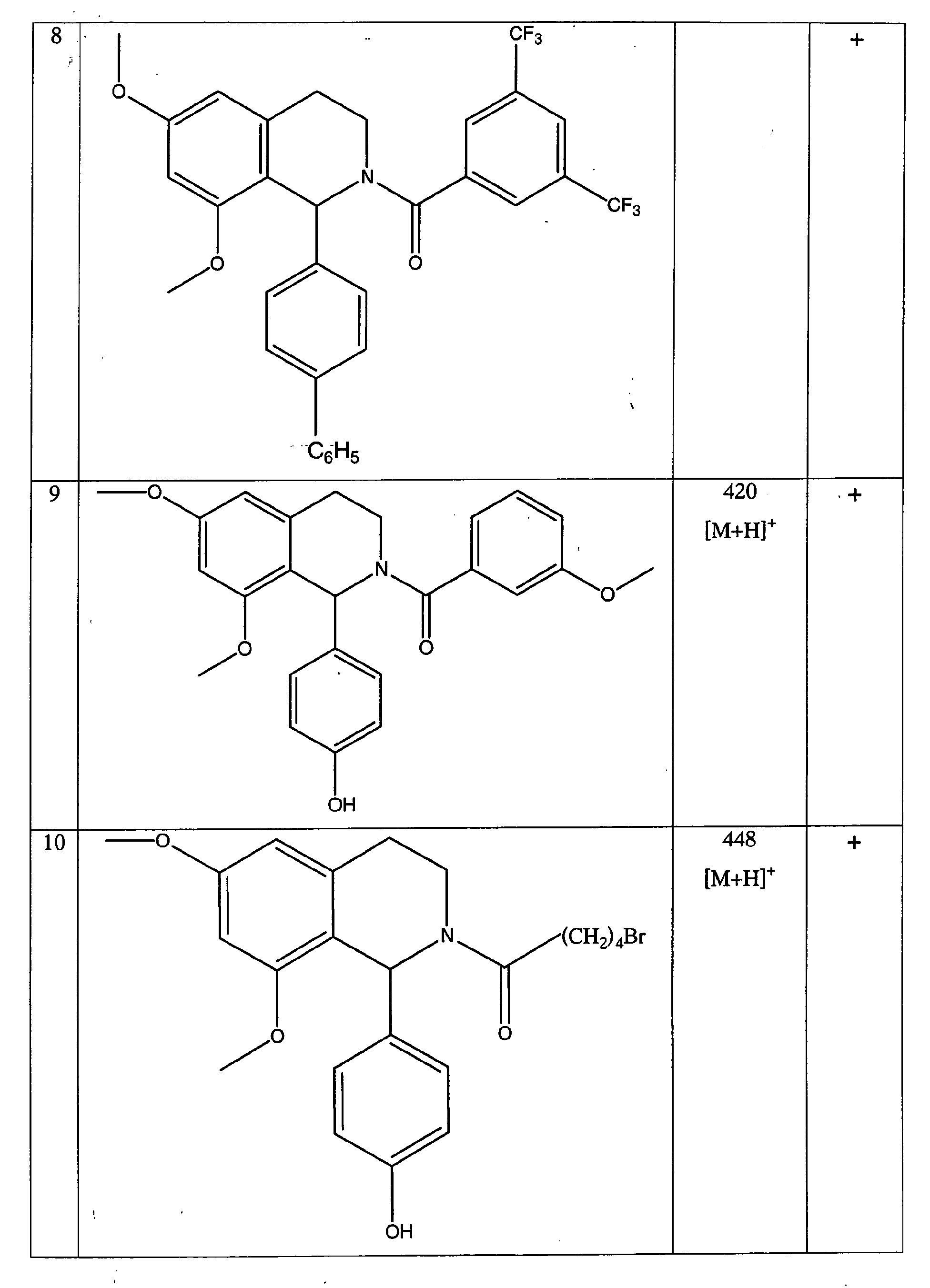

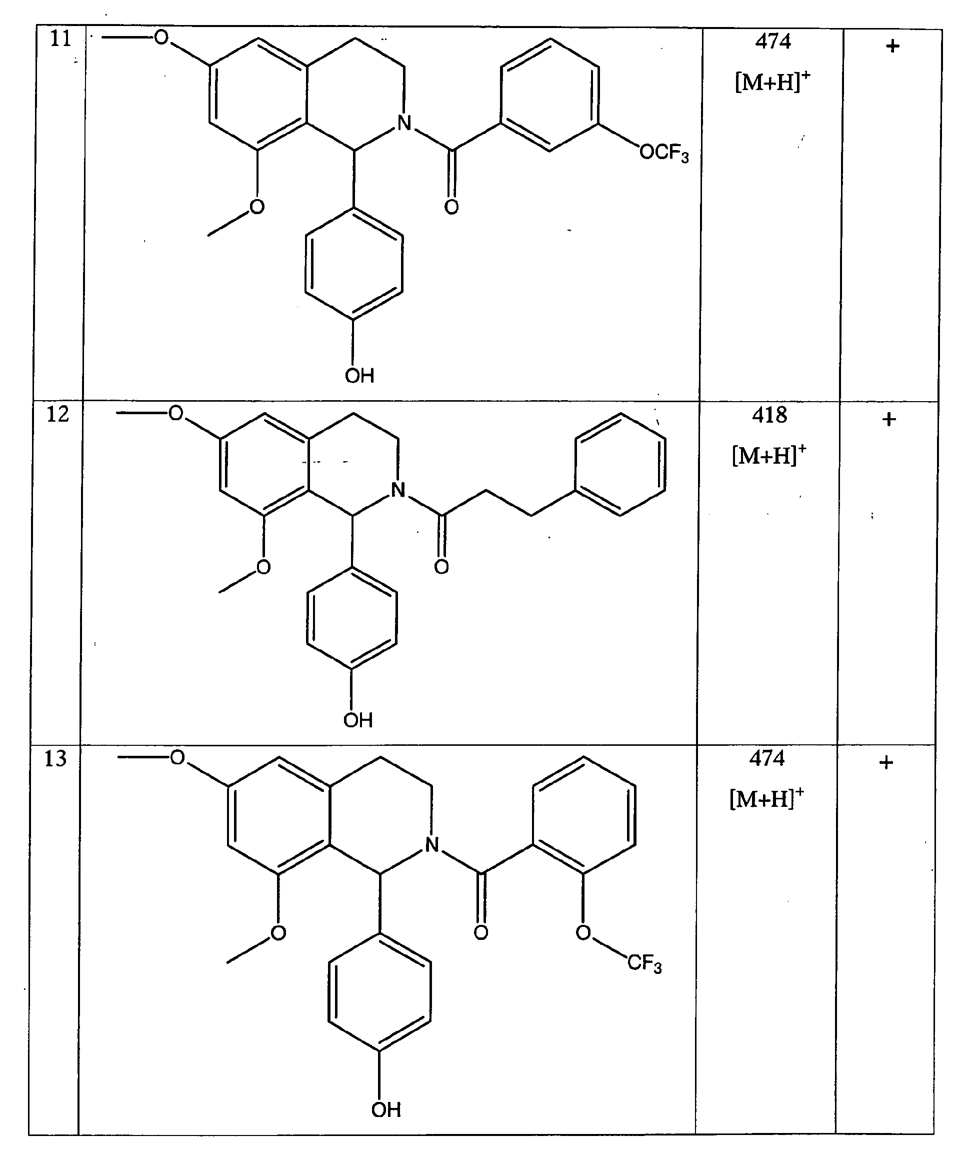

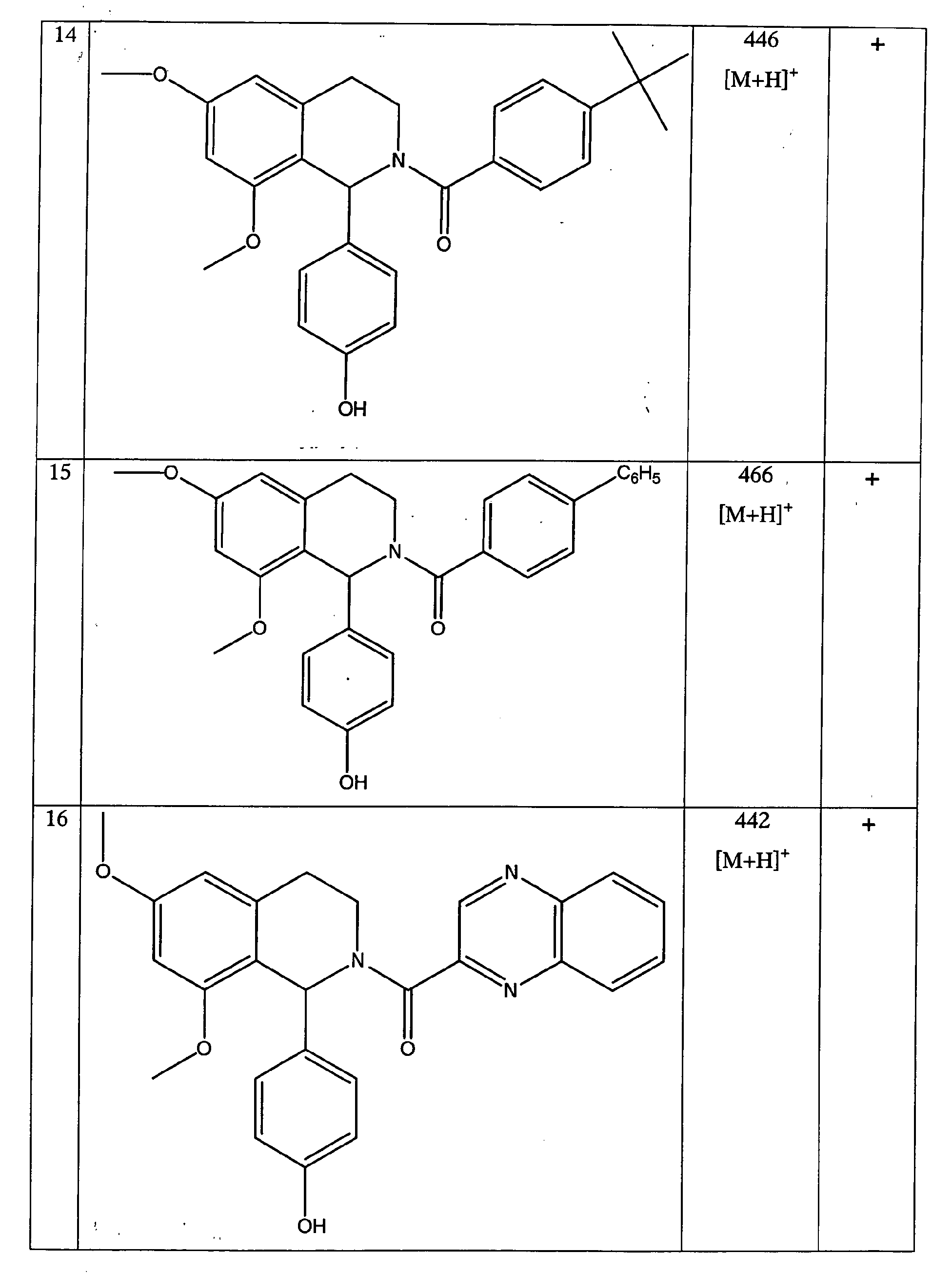

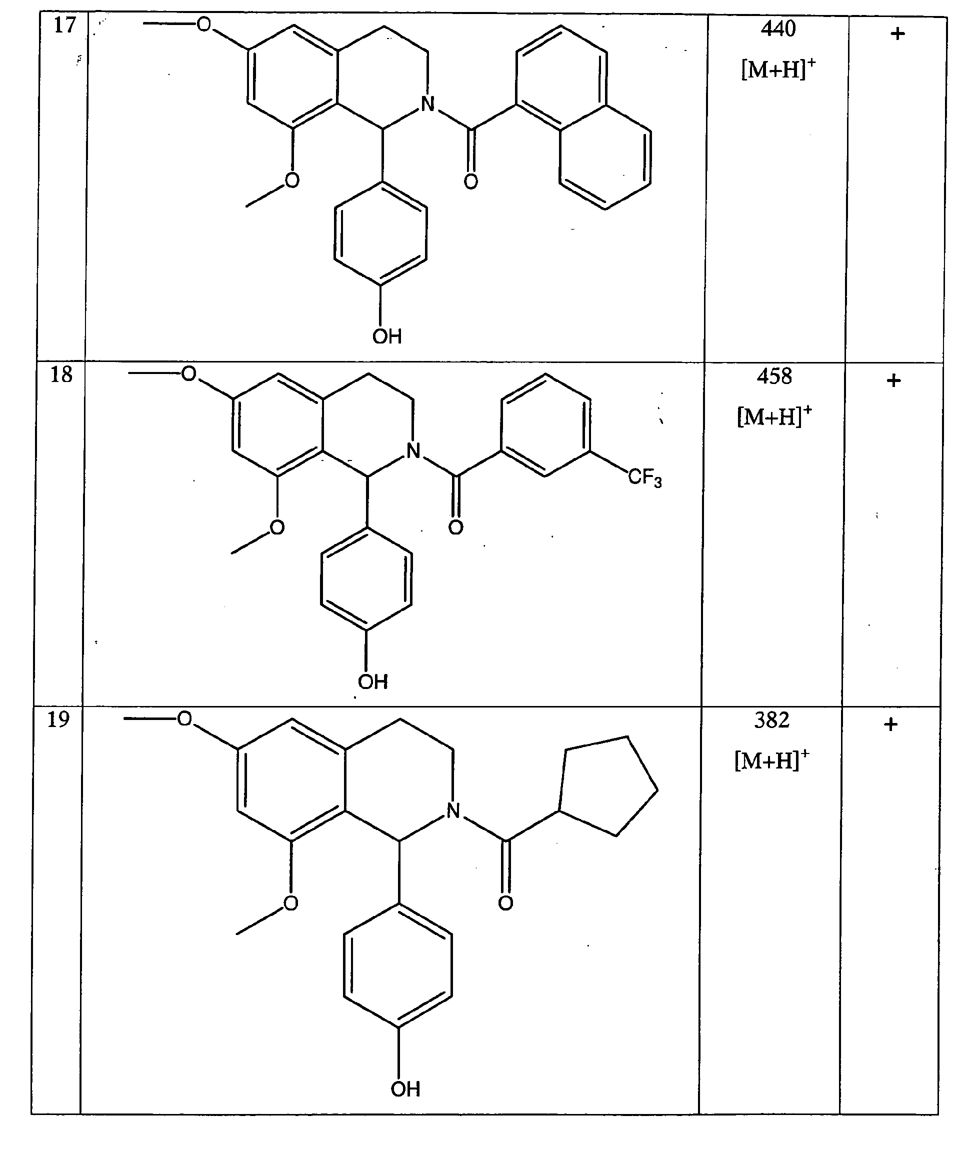

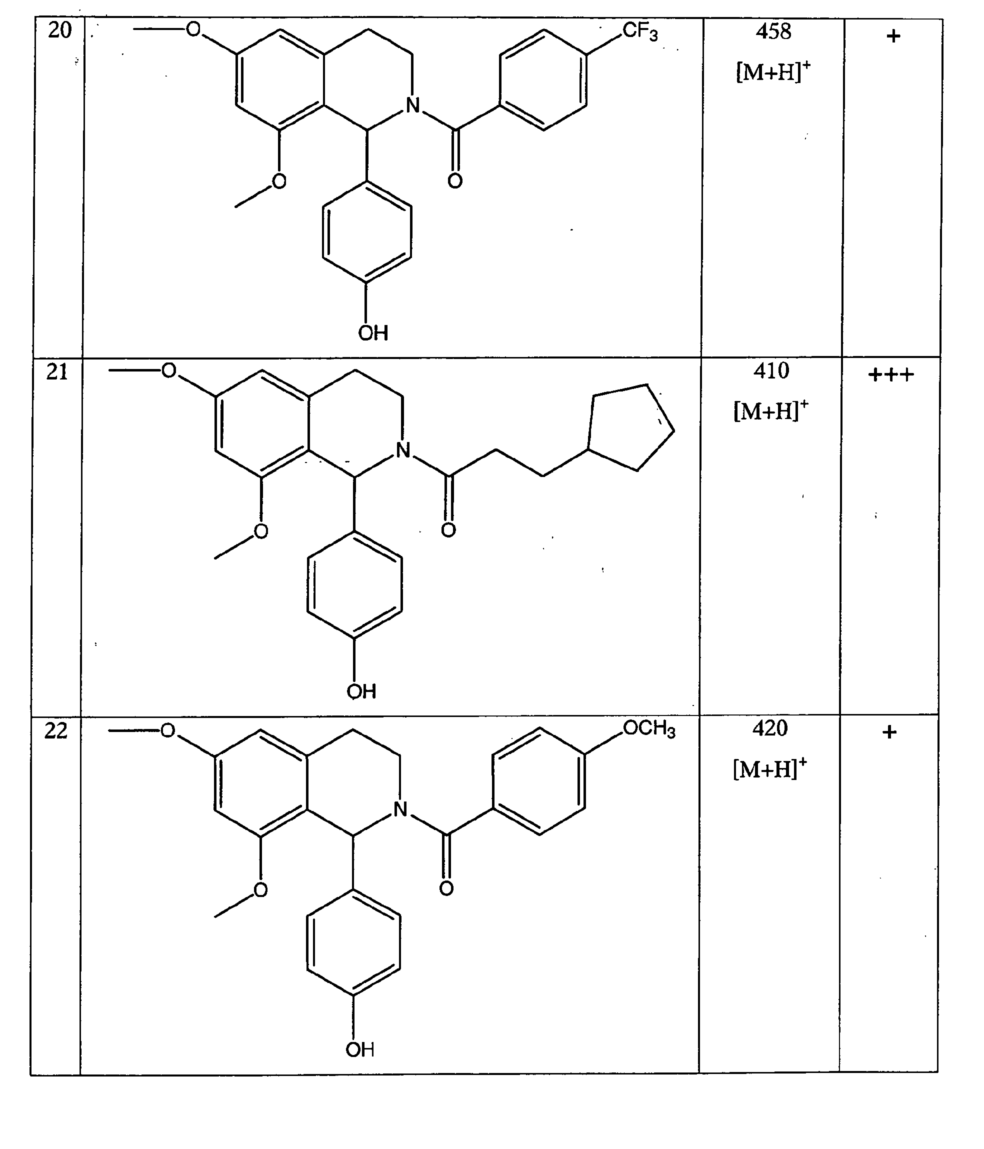

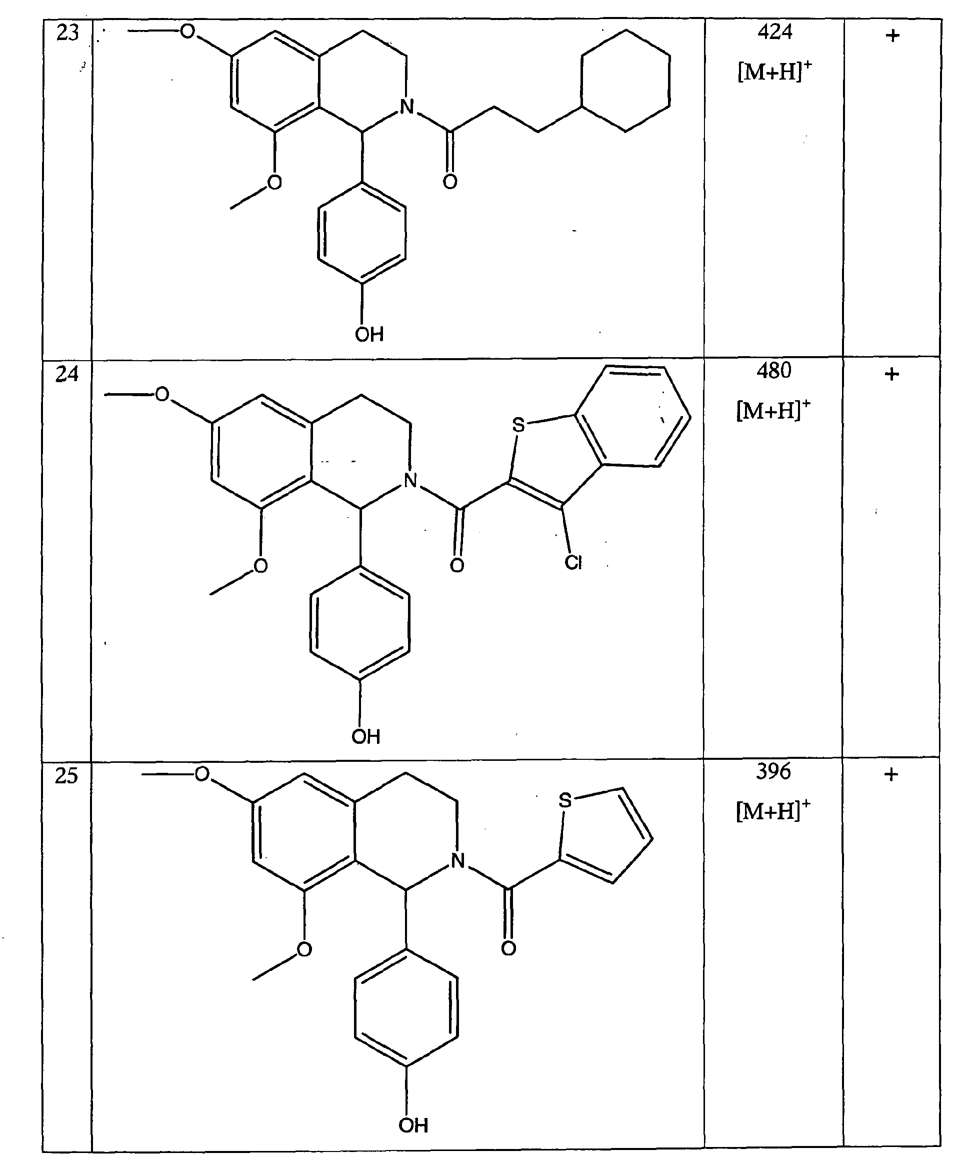

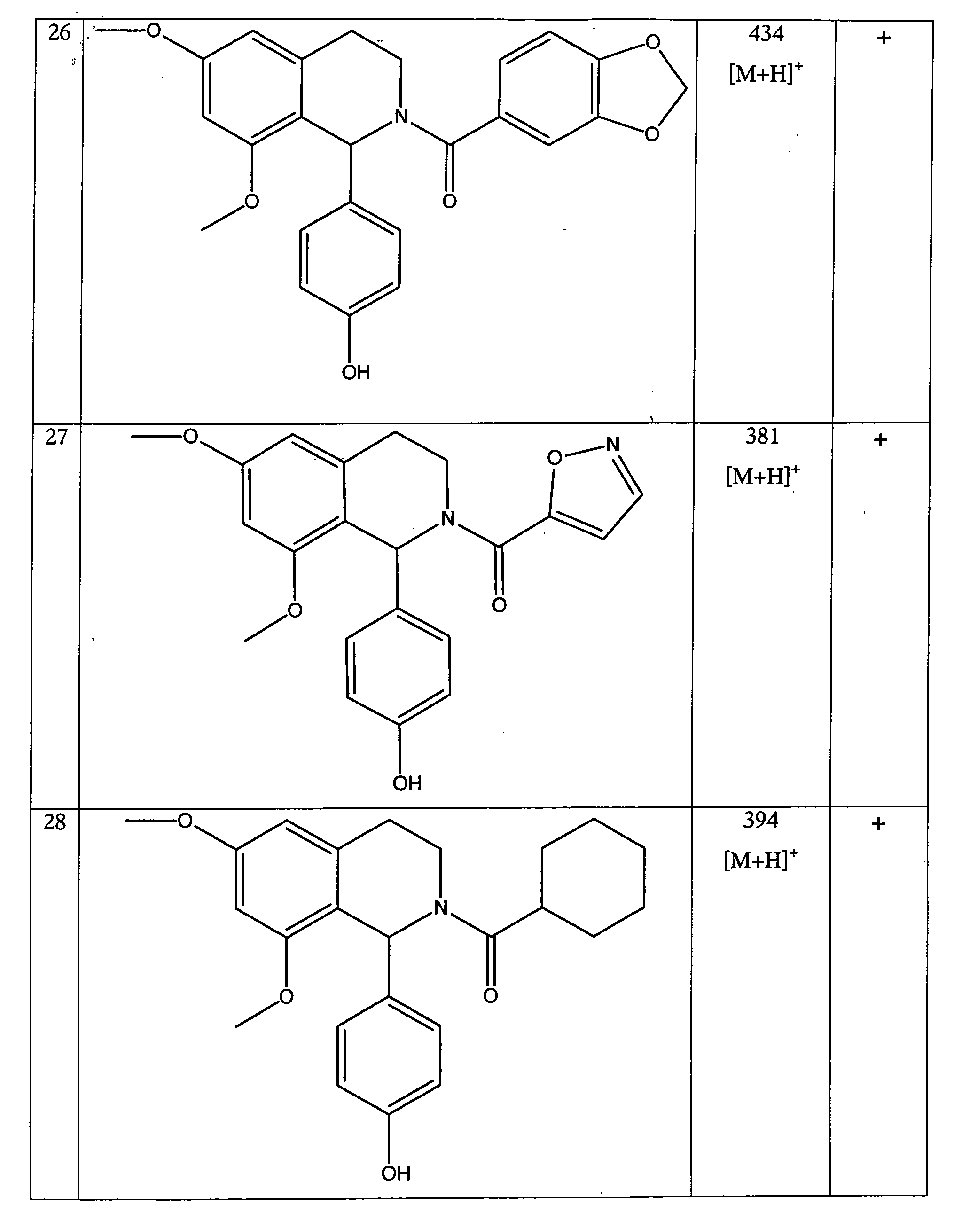

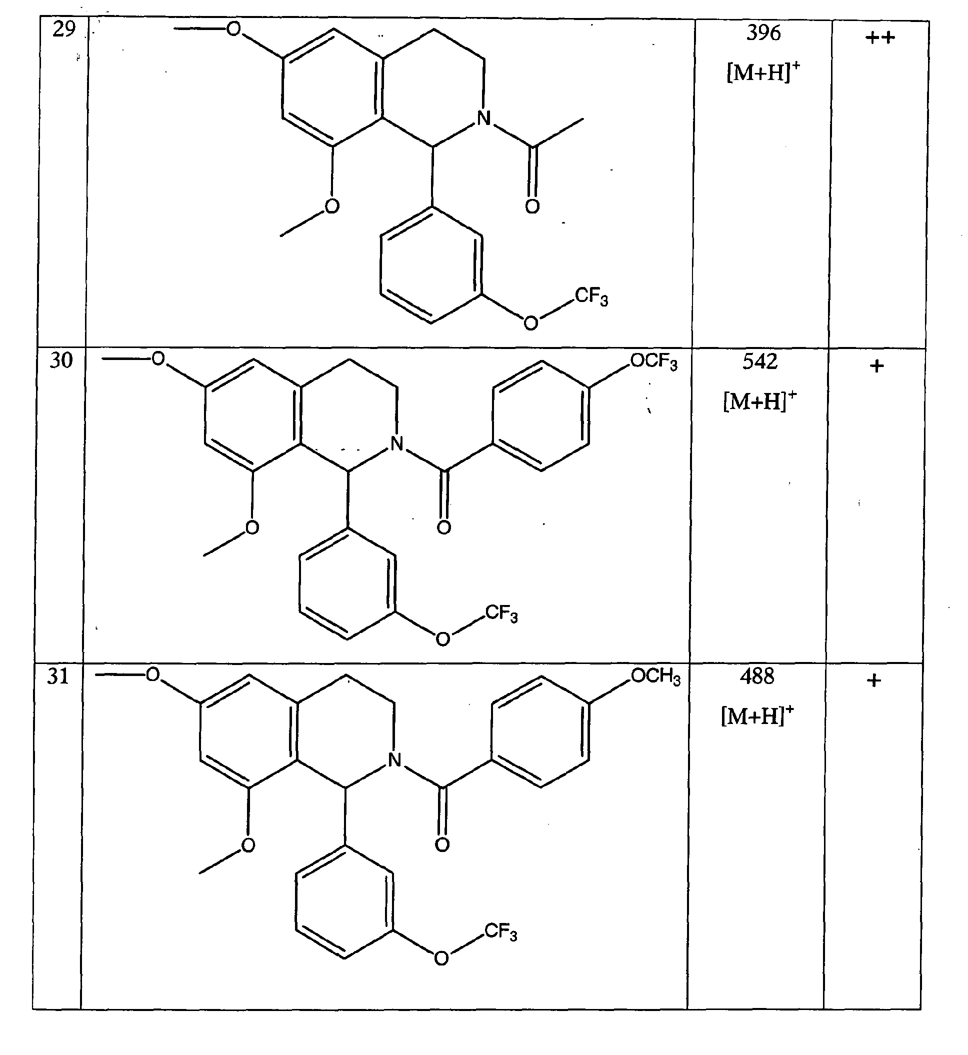

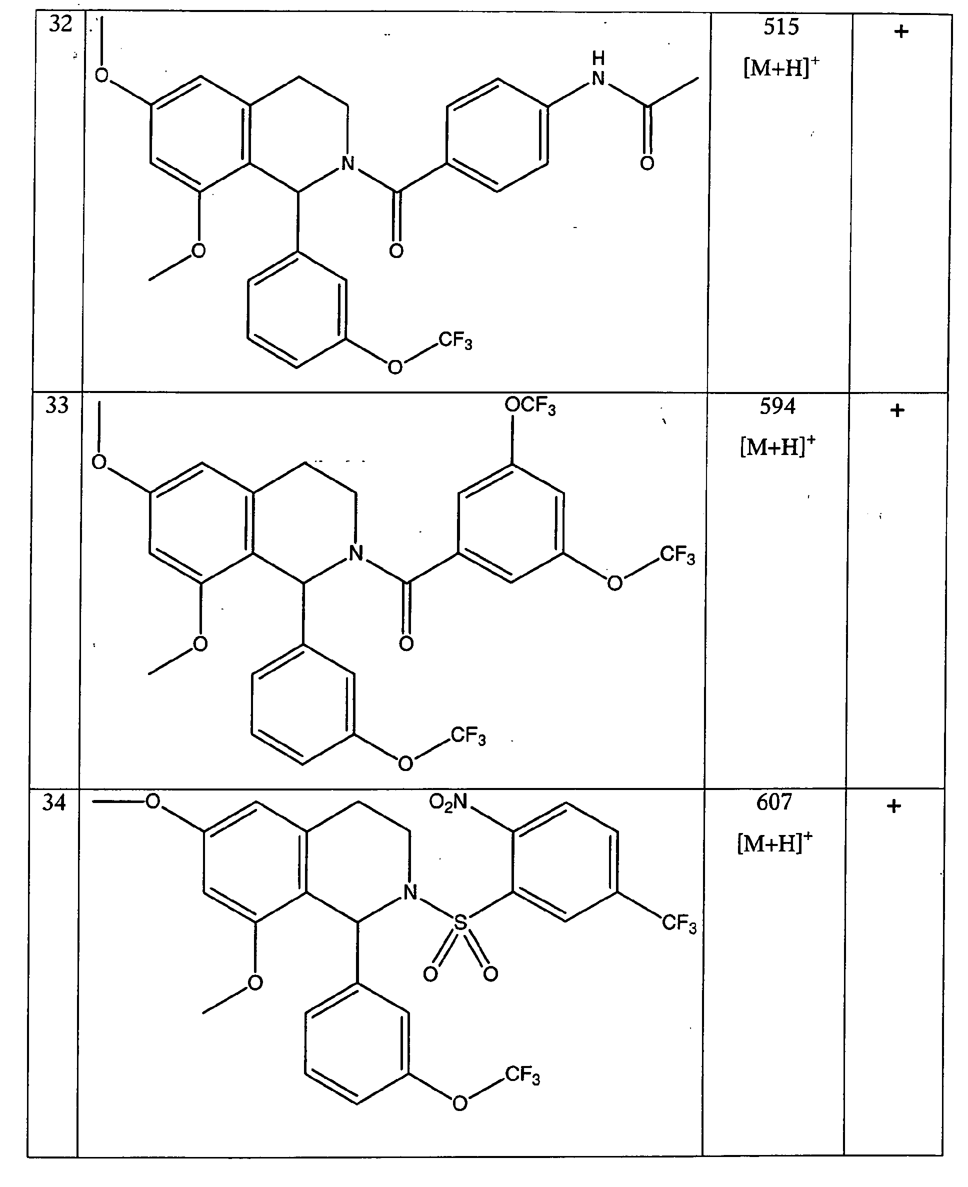

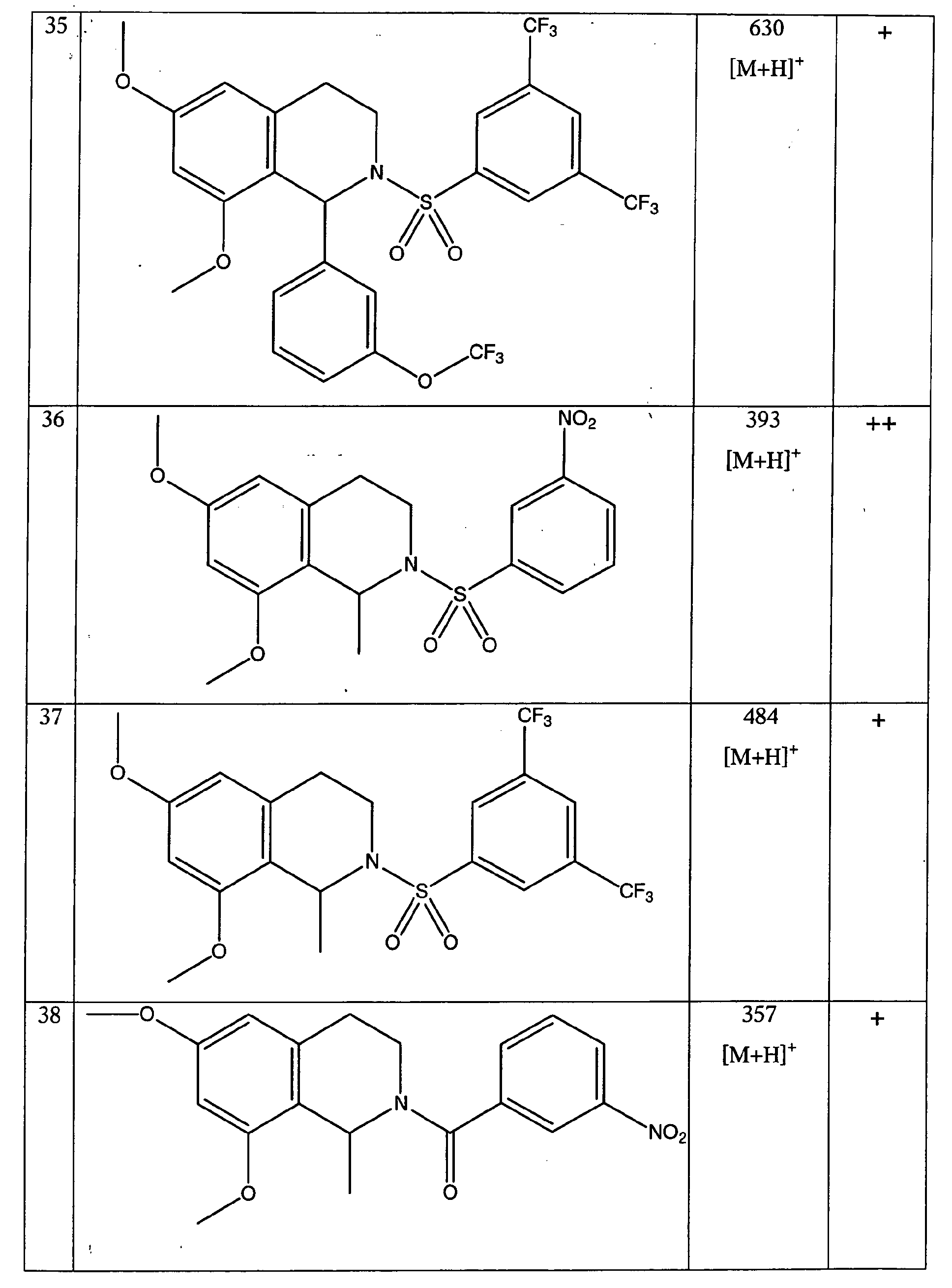

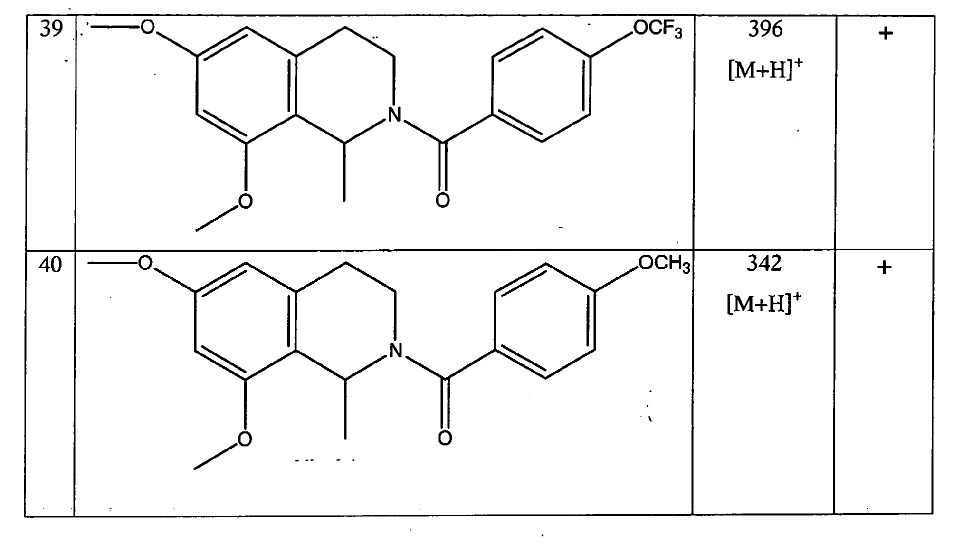

### NMR data on selected compounds:

### 3) (1-Biphenyl-4-yl-6,8-dimethoxy-3,4-dihydro-1H-isoquinolin-2-yl)-(3-bromo-phenyl) methanone

7.66 - 7.48 (m, 5H, ArH), 7.44 - 7.36 (m, 4H, ArH), 7.34 - 7.24 (m, 3H, ArH), 7.06 - 7.04 (m, 1H, ArH), 6.50 - 6.44 (m, 2H, ArH), 3.82 (s, 3H, OCH₃), 3.69 (s, 3H, OCH₃), 3.66 (s, 1H, CH), 3.58 - 3.48 (m, 1H, CH₂), 3.39 (m, 1H, CH₂), 3.08 - 2.72 (m, 2H, CH₂).

### 4) (1-Biphenyl-4-yl-6,8-dimethoxy-3,4-dihydro-1H-isoquinolin-2-yl)-(4-trifluoromethylphenyl)-methanone.

7.82 - 7.76 (m, 2H, ArH), 7.62 - 7.49 (m, 6H, ArH), 7.44 - 7.38 (m, 2H, ArH), 7.33 - 7.26 (m, 2H, ArH), 7.09 - 7.08 (m, 1H, ArH), 6.49 - 6.44 (m, 2H, ArH), 3.82 (s, 3H, OCH₃), 3.69 (s, 3H, OCH₃), 3.61 (s, 1H, CH), 3.50 - 3.43 (m, 1H, CH₂), 3.39 - 3.31 (m, 1H, CH₂), 3.00 - 2.92 (m, 1H, CH₂), 3.39 - 3.31 (m, 1H, CH₂), 2.80 - 2.70 (m, 1H, CH₂)

### 5) (1-Biphenyl-4-yl-6,8-dimethoxy-3,4-dihydro-1H-isoquinolin-2-yl)-(2-fluoro-4-trifluoromethyl-phenyl)-methanone.

7.64 - 7.53 (m, 6H, ArH), 7.44 - 7.38 (m, 2H, ArH), 7.34 - 7.26 (m, 3H, ArH), 7.11 (s, 1H, ArH), 6.50 - 6.42 (m, 2H, ArH), 3.83 (s, 3H, OCH₃), 3.70 (s, 3H, OCH₃), 3.61 (s, 1H, CH), 3.44 - 3.31 (m, 2H, CH₂), 2.99 - 2.91 (m, 1H, CH₂), 2.80 - 2.71 (m, 1H, CH₂).

### 6) (1-Biphenyl-4-yl-6,8-dimethoxy-3,4-dihydro-1H-isoquinolin-2-yl)-(4-tert-butylphenyl)-methanone.

7.61 - 7.49 (m, 6H, ArH), 7.44 - 7.25 (m, 6H, ArH), 7.09 - 7.07 (m, 1H, ArH), 6.48 - 6.43 (m, 2H, ArH), 3.82 (s, 3H, OCH₃), 3.68 (s, 3H, OCH₃), 3.35 - 3.31 (m, 1H, CH), 3.27 - 3.20 (m, 1H, CH₂), 3.05 - 2.90 (m, 1H, CH₂), 2.78 - 2.68 (m, 1H, CH₂), 1.34 (s, 9H, CH₃).

### 7) (1-Biphenyl-4-yl-6,8-dimethoxy-3,4-dihydro-1H-isoquinolin-2-yl)-(4-trifluoromethoxy-phenyl)-methanone.

7.61 - 7.49 (m, 5H, ArH), 7.44 - 7.35 (m, 4H, ArH), 7.33 - 7.26 (m, 2H, ArH), 7.24 - 7.19 (m, 1H, ArH), 7.09 - 7.07 (m, 1H, ArH), 6.48 - 6.43 (m, 2H, ArH), 3.82 (s, 3H, OCH₃), 3.68 (s, 3H, OCH₃), 3.62 (s, 1H, CH), 3.58 - 3.49 (m, 1H, CH₂), 3.39 - 3.31 (m, 1H, CH₂), 3.06 - 2.92 (m, 1H, CH₂), 2.79 - 2.70 (m, 1H, CH₂).

### 8) (1-Biphenyl-4-yl-6,8-dimethoxy-3,4-dihydro-1H-isoquinolin-2-yl)-(3,5-bis-trifluoromethyl-phenyl)-methanone.

8.13 - 8.10 (m, 1H, ArH), 8.02 - 7.98 (m, 2H, ArH), 7.62 - 7.50 (m, 3H, ArH), 7.44 - 7. 36 (s, 2H, ArH), 7.34 - 7.29 (m, 2H, ArH), 7.09 - 7.05 (m, 1H, ArH), 6.49 - 6.45 (m, 1H, ArH), 3.83 (s, 3H, OCH₃), 3.69 (s, 3H, OCH₃), 3.66 (s, 1H, CH), 3.50 - 3.35 (m, 2H, CH₂), 3.04 - 2.93 (m, 1H, CH₂), 2.84 - 2.75 (m, 1H, CH₂).

### 9) [1-(4-Hydroxy-phenyl)-6,8-dimethoxy-3,4-dihydro-1H-isoquinolin-2-yl]-(3-methoxyphenyl)-methanone.

7.54 - 7.47 (m, 1H, ArH), 7.19 - 7.14 (m, 1H, ArH), 7.09 - 7.01 (m, 4H, ArH), 6.95 (bs, 1H, OH), 6.85 - 6.80 (m, 2H, ArH), 6.63 - 6.55 (m, 2H, ArH), 3.93 (s, 6H, OCH₃), 3.81 (s, 3H, OCH₃), 3.71 (s, 1H, CH), 3.59 - 3.50 (m, 2H, CH₂), 3.30 - 3.18 (m, 1H, CH₂), 2.88 - 2.78 (m, 1H, CH₂).

### 14) (4-tert-Butyl-phenyl)-[1-(4-hydroxy-phenyl)-6,8-dimethoxy-3,4-dihydro-1H-isoquinolin-2-yl]-methanone.

9.37 (s, 1H. OH), 7.48 (q, 4H, ArH), 6.89 (q, 4H, ArH), 6.59 - 6.51 (m, 2H, ArH), 3.89 (s, 3H, OCH₃), 3.77 (s, 3H, OCH₃), 3.64 - 3.57 (m, 1H, CH₂), 3.55 - 3.51 (m, 1H, CH₂), 3.24 - 3.16 (m, 1H, CH₂), 2.84 - 2.74 (m, 1H, CH₂), 1.41 (s, 9H, CH₃).

### 20) [1-(4-Hydroxy-phenyl)-6,8-dimethoxy-3,4-dihydro-1H-isoquinolin-2-yl]-(4-trifluoromethyl-phenyl)-methanone

9.07 (s, 1H, OH), 7.48 (q, 4H, ArH), 6.58 (q, 4H, ArH), 6.23 (q, 2H, ArH), 3.56 (s, 3H, OCH₃), 3.44 (s, 3H, OCH₃), 3.32 (s, 1H, CH), 3.22 - 3.15 (m, 1H, CH₂), 2.93 - 2.84 (m, 1H, CH₂), 2.73 - 2.61 (m, 1H, CH₂), 2.50 - 2.40 (m, 1H, CH₂).

### 29) 1-[6,8-Dimethoxy-1-(3-trifluoromethoxy-phenyl)-3,4-dihydro-1H-isoquinolin-2-yl]-ethanone.

7.32 (t, 1H, ArH), 7.10 (d, 2H, ArH), 6.91 (s, 1H, ArH), 6.47 - 6. 42 (m, 2H, ArH), 3.79 (s, 3H, OCH₃), 3.77 (s, 1H, CH), 3.67 (s, 3H, OCH₃), 3.40 - 3.33 (m, 1H, CH₂), 3.27 - 3.13 (m, 1H, CH₂), 3.03 - 2.89 (m, 1H, CH₂), 2.83 - 2.72 (m, 1H, CH₂).

### 36) 6,8-Dimethoxy-1-methyl-2-(3-nitro-benzenesulfonyl)-1,2,3,4-tetrahydro-isoquinoline.

8.38 (dd, 1H, ArH), 8.29 (t, 1H, ArH), 8.17 (d, 1H, ArH), 7.78 (t, 1H, ArH), 6.30 (s, 1H, ArH), 6.09 (s, 1H, ArH), 5.10 (q, 1H, CH), 3.78 - 3.71 (m, 1H, CH₂), 3.68 (s, 3H, OCH₃), 3.63 (s, 3H, OCH₃), 3.58 - 3.48 (m, 1H, CH₂), 2.59 - 2.49 (m, 2H, CH₂), 1.28 (d, 3H, CH₃).

### Biological activity

The large conductance, voltage-dependent and Ca²⁺-activated potassium channel BK is a potassium selective ion channel and belongs to the subfamily of K_{Ca} channels. Four BK alpha-subunits form a functional channel that can be regulated by intracellular Ca²⁺ concentration, membrane voltage, and other mechanisms like phosphorylation states or beta subunits. To test the biological activity of the compounds, we applied two different techniques, a fluorescence based assay using a voltage sensitive dye (Eₘ-Assay) as well as exploiting electrophysiological methods.

### Eₘ-Assay:

CHO cells permanently transfected with cloned *hSlo* (*α-hslo* and *β-bSlo*), yielding typical BK potassium currents (Zhou *et al.*, *Pflügers Arch.,* 436: 725-734 (1998), were used for the evaluation of compound activity. Activation or inhibition of BK channels in these cells leads to a change of the electrochemical gradient causing a hyperpolarized or depolarised membrane potential, respectively.

To determine changes in the membrane potential of the cells we used the voltage sensitive dye DiBAC₍₄₎3 (Molecular Probes) in a kinetic assay system using a fluorescent plate reader (Manning and Sontheimer, *J*. *Neurosci. Meth.*, 91: 73-81 (1999). The anionic bis-oxonol DiBAC₍₄₎3 is a voltage sensitive dye which partitions from the extracellular environment into the cell where it reversibly binds to intracellular proteins, a kinetic process depending on the membrane potential of the cell. At depolarised potentials (i.e. at a reduced K⁺ efflux due to blocked K⁺ channels) the dye accumulates in the cell leading to an increased fluorescence intensity, due to its increased fluorescence if bound to cellular proteins. At hyperpolarized potentials (i.e. at an increased K⁺ efflux due to the opening of K⁺ channels), the dye partitions out of the cell causing a decreased fluorescence intensity.

*hSlo* transfected CHO cells where maintained in DMEM supplemented with 10% FCS, 250µg/ml Geneticin, 100µg/ml Hygromycin, 1xHT-Supplement, and 1xNon-essential Amino Acids and cultured in a humidified CO₂ incubator. After trypsination, cells where plated with a density of 5x10⁴ cells per well on a clear 96-well plate and incubated for 24h. Cells where washed once with PBS, once with PBS containing 20mM HEPES (adjusted to pH 7.4 with NaOH) and 2µM DiBAC₍₄₎3 (DPBS-DiBAC solution). 180µl of the dPBS-DiBAC solution was then added to the cells and the plate incubated for 30-60 min at 37°C. During this time the dye could partition into the cells and reach a certain steady-state distribution, depending on the resting membrane potential. Test and reference compounds were stored as DMSO stock solutions and diluted in dPBS-DiBAC solution to the desired concentration.

Fluorescence intensity (Ex.: 485nm/Em.: 520nm) of each well was detected in the plate reader (Fluostar, BMG) every 60 seconds. After recording the baseline fluorescence for 7 minutes, 20µl test- and reference compounds were added and the fluorescence intensity was detected for additional 15 minutes. Background was subtracted, data values were normalized and expressed as a change in fluorescence intensity against time. The change in fluorescence intensity caused by the test compounds was evaluated, compared to the effect of the reference compound NS004, and the ratio was determined (see Table I).

### Electrophysiological studies:

CHO cells permanently transfected with cloned *α-hSlo* and *β-bSlo* were maintained as described above and used for electrophysiological characterisation. The whole-cell configuration of the patch-clamp technique was used to determine the effect of modulators on BK currents in these cells. The cell line expressing functional BK currents (Zhou et al., *Pflügers Arch.* 436, p.725 (1998)) were plated onto glass cover slips with a density of 1-5x10⁴ cells/cover slip, incubated (37°C, 5% CO₂) and used for patch-clamp experiments within 24-48 h. Cells were bathed in mammalian ringer solutions containing (in mM): 160NaCl, 4.5KCl, 2CaCl₂, 1MgCl₂, 10HEPES, adjusted to pH 7.4, 290-310 mOsm. The internal pipette solution contained (in mM): 160KCl, 2CaCl₂, 1MgCl₂, 10 HEPES, EGTA was added to reach a free [Ca²⁺]ᵢₙₜₑᵣₙₐₗ = 1×10⁻⁶M, adjusted to pH 7.2, 290-310 mOsm. Borosilicate pipettes with a resistance of 2-3 MΩ were filled with the internal solution and mounted on an appropriate holder. Prior to measurements a recording chamber was mounted onto the cell-plated cover slips and the cells were perfused with a simple syringe driven perfusion system. Compounds were added in the final concentration (2x10⁵M) to the bath solution using the same system. An EPC-9 patch-clamp amplifier with Pulse and PulseFit software (HEKA) was used to record and analyze currents.

After addition of the compounds to the bath solution their modulating effect was determined by the increase or decrease of specific BK currents after reaching steady-state relative to the BK current before application of drugs (see Table II).

**Table II:**

| Results from the electrophysiological studies are given as the ratio of current increase after application of compound (20µM) relative to the control current before compound application. Currents were determined after reaching steady-sate. Ranges are < 1.2 = +, > 1.2 = ++ | | |
|---|---|---|
| **Compound #** | **Mass** | **Effect** |
| 30 | 542 | + |
| 36 | 393 | ++ |

## Claims

1. A compound of the general Formula (I), a salt, a physiologically functional derivative, or a prodrug thereof, 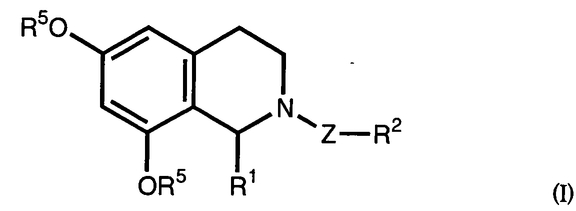 wherein
Z is carbonyl or sulfonyl;
R¹ is alkyl, alkenyl, alkynyl, aryl, H, halogen, haloalkyl, haloalkoxy, hydroxyalkyl, cycloalkyl or heteroaryl;
R² is -CH₂-SO₂-alkyl, -CH₂-SO₂-cycloalkyl, -CH₂-SO₂-aryl, -CH₂-SO₂-heteroaryl, aryl, haloalkyl, haloalkoxy, hydroxyalkyl, cycloalkyl, heteroaryl or a linear or branched C₂-C₆-alkyl group, which is optionally substituted by one or more substituents R³;
R³ is H, alkyl, alkenyl, alkynyl, cycloalkyl, -CO₂R⁴, -CONHR⁴, -CR⁴O, -SO₂R⁴, -NR⁴-CO-R⁴, alkoxy, alkylthio, -OH, -SH, -O-aryl, -O-cycloalkyl, -S-aryl, -S-cycloalkyl, hydroxyalkyl, halogen, haloalkyl, haloalkoxy, CN, NO₂, hydroxyalkylamine, aminoalkyl, alkylamine, aryl or heteroaryl;
R⁴ is H, halogen, alkyl, alkenyl, alkynyl, cycloalkyl, alkoxy, alkylthio, -O-aryl, -O-cycloalkyl, OH, SH, -S-aryl, -S-cycloalkyl, hydroxyalkyl, haloalkyl, haloalkoxy, hydroxyalkylamine, aminoalkyl, alkylamine, aryl or heteroaryl;
R⁵ independently represents alkyl, alkenyl, or alkynyl;
wherein
a C₂-C₆-alkyl group denotes a linear or branched C₂-C₆-alkyl group, which is optionally substituted by one or more substituents R³, R³ being as defined above;
an alkyl group, if not stated otherwise, denotes a linear or branched C₁-C₁₂-alkyl group, which is optionally substituted by one or more substituents R³, R³ being as defined above;
an alkenyl group, if not stated otherwise, denotes a linear or branched C₁-C₁₂-alkenyl group, which is optionally substituted by one or more substituents R³, R³ being as defined above;
an alkynyl group, if not stated otherwise, denotes or a linear or branched C₁-C₁₂-alkynyl group, which is optionally substituted by one or more substituents R³, R³ being as defined above;
a cycloalkyl group denotes a non-aromatic ring system containing three to eight carbon atoms, wherein one or more of the carbon atoms in the ring can be substituted by a group X, wherein X is selected from the group consisting of N, S, O, SO, SO₂, NR⁴ and CO; the cycloalkyl group is optionally substituted by one or more substituents R³, R³ being as defined above;
an alkoxy group denotes an O-alkyl group, the alkyl group being as defined above;
an haloalkyl group denotes an alkyl group which is substituted by one to five halogen atoms, the alkyl group being as defined above;
a hydroxyalkyl group denotes an HO-alkyl group, the alkyl group being as defined above;
an haloalkyloxy group denotes an alkoxy group which is substituted by one to five halogen atoms, the alkyl group being as defined above;
a hydroxyalkylamino group denotes an (HO-alkyl)₂-N- group or HO-alkyl-NH- group, the alkyl group being as defined above;
a halogen atom is chlorine, bromine, fluorine or iodine;
an aryl group denotes an aromatic group having five to fifteen carbon atoms and is optionally substituted by one or more substituents R³, R³ being as defined above;
a heteroaryl group denotes a 5- or 6-membered heterocyclic group which contains at least one heteroatom selected from the group consisting of O, N, and S, which can be fused to another ring and which is optionally substituted by one or more substituents R³, R³ being as defined above;
in the CH₂-SO₂-alkyl group, the alkyl group is as defined above;
in the CH₂-SO₂-cycloalkyl group, the cycloalkyl group is as defined above;
in the CH₂-SO₂-aryl group, the aryl group is as defined above;
in the CH₂-SO₂-heteroaryl group, the heteroaryl group is as defined above.

2. The compound of claim 1, wherein R¹ is a phenyl group which is optionally substituted with one or more substituents R³.

3. The compound of claim 1, wherein R¹ = methyl.

4. The compound of claim 1, wherein Z = CO and R¹ is a phenyl group which is optionally substituted with one or more substituents R³.

5. The compound of claim 1, wherein Z = SO₂, R¹ = methyl.

6. A pharmaceutical composition comprising a compound as defined in any one of claims 1 to 5, in free form or in the form of pharmaceutically aceptable salts or physiologically functional derivatives, and a pharmaceutically acceptable diluent or carrier.

7. A compound according to any one of claims 1 to 5 for the use as a medicament.

8. The use of a compound according to any one of claims 1 to 5, for the preparation of a medicament for the prevention, alleviation or treatment of diseases, conditions or disorders which are associated with, or dependent on the membrane potential or conductance of cells in mammals, including a human.

9. The use according to claim 8 wherein the diseases are asthma, cystic fibrosis, obstructive pulmonary disease, convulsions, vascular spasms, urinary incontinence, urinary instability, urinary urgency, bladder spasms, ischemia, cerebral ischemia, traumatic brain injury, neurodegeneration, migraine, pain, psychosis, hypertension, epilepsy, memory and attention deficits, functional bowel disorders, erectile dysfunction, female sexual dysfunction, immune suppression, autoimmune disorders, dysfunction of cellular proliferation, diabetes, premature labour, or other disorders associated with or responsive to the modulation of potassium channels.

10. A process for the preparation of a compound as defined in claim 1 which comprises the step of reacting a chloride of Formula (VI) with an tetrahydroisoquinoline of Formula (V)
